# EUROPEAN PATENT APPLICATION

(11) **EP 4 289 864 A1**
(43) Date of publication of application: **13.12.2023**
(21) Application number: 22748979.6
(22) Date of filing: 26.01.2022
(51) Int. Cl.: C07K 16/28, C12N 5/10, A61K 39/395

(54) **METHOD FOR INHIBITING TUMOUR CELL GROWTH BASED ON CCDC112**

(30) Priority: 08.02.2021 CN 202110172875
(71) Applicant: BIOTROY THERAPEUTICS, Shanghai 201413 (CN)
(72) Inventor: XU, Jie, Shanghai 201413 (CN); SUN, Yufan, Shanghai 201413 (CN); ZHAO, Xuehua, Shanghai 201413 (CN)
(74) Representative: Weickmann & Weickmann PartmbB
(86) International application number: PCT/CN2022/073951
(87) International publication number: WO 2022/166700

(57) **Abstract**

The application of CCDC112 in tumor inhibition promotes the killing effect of immune cells on tumor cells by means of inhibiting the function or activity of CCDC112. CCDC112 has application value in the preparation of anti-tumor drugs and companion diagnostic markers.

## Description

### CROSS-REFERENCE TO RELATED APPLICATIONS

The invention claims priority to Chinese Patent Application No. 202110172875.X, entitled "Method for Inhibiting Tumor Cell Growth Based on CCDC112" and filed with the China National Intellectual Property Administration on February 8, 2021, the entire contents of which are incorporated herein by reference.

### TECHNICAL FIELD

The invention relates to the technical field of biomedicine, in particular to a method for inhibiting tumor cell growth based on CCDC112 and its use.

### BACKGROUND OF THE INVENTION

There is a lack of effective methods for the treatment of advanced recurrent tumors, and alternative or improved solutions with better effects are needed in this technical field. Immunotherapy of tumors has undergone important developments in recent years. The immune negative checkpoint regulator (NCR) pathway has proven to be an excellent clinical target for the treatment of human immune-related diseases. Using monoclonal antibodies (mAbs) to block two NCRs, CTLA-4 and PD-1, to enhance tumor immunity is revolutionizing the treatment of cancer, and these pathways have been identified as clinically validated targets in the context of human diseases. More recently, soluble forms of NCR ligands that trigger the NCR pathway have entered clinical trials as immunosuppressive drugs for the treatment of autoimmunity (i.e., AMP-110/B7-H4-Ig for rheumatoid arthritis). It is worth noting that the effective rate of immune checkpoint blockade therapy is relatively low, and long-term treatment will lose efficacy in patients who were initially treated effectively. This suggests that there may be important alternative mechanisms and pathways for tumor immune escape. To treat cancer more effectively, novel tumor immune molecular targets and treatment method must be identified.

Recent studies have identified the inhibitory leukocyte immunoglobulin-like receptor subfamily B (LILRB) and the related immunoreceptor tyrosine-based inhibitory motif (ITIM)-containing receptor LAIR1 have tumor-promoting functions in a variety of hematopoietic and solid cancer cells. ITIM-containing receptors are expressed on a variety of immune cells and signal through the recruitment of the phosphatases SHP-1, SHP-2, or SHIP, resulting in negative regulation of immune cell activation. Similar to CTLA-4 and PD-1, LILRB is considered an immune checkpoint factor. LILRB2 is expressed on monocytes, macrophages, and dendritic cells and can inhibit innate immunity in a cell-autonomous manner and T cell activation through indirect mechanisms. Previous studies have reported that antagonists of LILRB2 can reduce the local infiltration of myeloid-derived suppressor cells (MDSCs) and regulatory T cells (Treg) in tumors and promote antitumor immune responses (Hui-Ming Chen et al, J Clin Invest. 2018;128(12):5647-5662). This indicates that LILRB2 can inhibit immune cells through MDSC and Treg after binding its ligand and participate in the process of tumor immune escape. LILRB4 is similar to LILRB2 in structure and function, and LILRB4 was reported to be expressed on the cell surface of MDSCs (Pauline de Goeje, et al., Oncoimmunology, 19 Mar 2015, 4(7): e1014242). Its ligands include APOE (Cancer Immunol Res. 2019 Aug; 7(8):1244-1257) and blocking the binding of APOE to LILRB4.

Lymphotoxin-beta receptor (LTBR), also known as tumor necrosis factor receptor superfamily member 3 (TNFRSF3), tumor necrosis factor receptor type III (TNF-RIII), is a single-pass type-I membrane protein containing four TNFR-Cys repeat regions. In addition to interacting with HCV core protein, LTBR binds not only to itself but also to TRAF3, TRAF4, and TRAF5. Previous studies reported that the knockout of LTBR in mice caused the decrease of MDSCs, suggesting that LTBR may play an important role in the induction and maintenance of MDSCs (Anja K Wege et al, Innate Immun. 2014 Jul;20(5):461-70). At present, the known ligand of LTBR is lymphotoxin, and no other ligands have been reported yet.

The CCDC protein is a protein domain that exists in a variety of natural proteins. At least 200 CCDC proteins have been discovered so far. Different CCDC proteins have diverse biological functions, including participating in intercellular transmembrane signal transduction, genetic signal transcription, promoting cell proliferation and apoptosis, and regulating the invasion and metastasis of malignant tumor cells and other biological behaviors. The full name of the CCDC112 gene is "Coiled-coil domain-containing protein 112", and its function has not been disclosed, nor has there been any report on the inhibition of tumors based on CCDC112. Therefore, further seeking novel markers that can be used for inhibiting tumors or tumor cells is a continuous demand in the art.

The invention is provided in view of this.

### BRIEF SUMMARY OF THE INVENTION

The primary objective of the invention is to seek a novel tumor immune molecular target and treatment method.

For achieving above-mentioned objective of the invention, the technical solution that the invention adopts is as follows:

In one aspect, the invention provides a method for regulating tumor cells in vitro or in vivo, including the following step:
a) for tumor cells expressing CCDC112, altering the function or activity of CCDC112; wherein
   preferably, the regulating is for inhibiting and the altering is for inhibiting.
   Further, the method may further include step b):
b) contacting the tumor cells with immune cells; wherein step b) can be prior to or subsequent to step a).

In some embodiments, the inhibition can be at a protein level or at a gene level.

Preferably, the methods for the inhibition include but are not limited to any one of:
i. performing gene editing on the CCDC112 gene;
ii. interfering with the expression of the CCDC112 gene; and
iii. inhibiting the function or activity of the CCDC112 protein by using an antibody.

The above-mentioned inhibition results in an effect on binding of CCDC112 to any one of the receptors LILRB2, LTBR, LILRB4 and CD30; preferably, the effect is to inhibit or block the binding of CCDC112 to any one of the receptors LILRB2, LTBR, LILRB4 and CD30; preferably, the CCDC112 is expressed in the tumor cells, and LILRB2, LILRB4, LTBR and CD30 are expressed in the immune cells.

Further, the tumor cells and immune cells can be tumor cells and immune cells isolated in vitro, or tumor cells and immune cells existing in vivo.

In one aspect, the invention further provides a method for regulating a ratio of MDSCs or Treg cells in vitro or in vivo, wherein the method includes the step of altering the function or activity of CCDC112 in the cells.

In one aspect, the invention further provides a method for regulating the activity of LILRB2, LTBR, LILRB4 or CD30 in cells in vitro or in vivo, wherein the method includes the step of altering the function or activity of CCDC112 in cells.

In one aspect, the invention further provides a method for screening a drug or reagent for preventing or treating a tumor, wherein by screening an inhibitor or antibody that inhibits interaction between CCDC112 and a receptor LILRB2, LILRB4, LTBR or CD30, a candidate drug or agent can be obtained.

In one aspect, the invention further provides a method for screening or identifying an inhibitory molecule or an inhibitor, wherein the method includes any one or more of:
a) analyzing the effect of the candidate molecule on the binding of CCDC112 to the receptor LILRB2, LTBR, LILRB4 or CD30;
b) analyzing the effect of the candidate molecule on myeloid-derived suppressor cells (MDSCs);
c) analyzing the effect of the candidate molecule on Treg cells; and
d) analyzing the effect of the candidate molecule on immune cells killing tumor cells;

wherein the candidate molecule is a CCDC112 inhibitory molecule;
preferably, the inhibitory molecules or inhibitor is a tumor suppressor molecule or inhibitor.

More preferably, the inhibitory molecule or inhibitor is an antibody or antigen-binding fragment.

In one aspect, the invention also provides a method for preparing an antibody, wherein the method includes:
a) a step of preparing antibodies against CCDC112; and:
b) screening an antibody having an effect on the binding of CCDC112 to LILRB2, LTBR, LILRB4, or CD30.

Preferably, in step a), the antibody is prepared by injecting the CCDC112 protein as an immunogen to subjects, such as mice, or screening a natural library. In an example, the CCDC112 protein has an amino acid sequence as set forth in SEQ ID NO: 10.

In one aspect, the invention further provides an agonistic or antagonistic compound, wherein the compound can regulate the interaction between CCDC112 and any one of LILRB2, LILRB4, LTBR and CD30.

Preferably, the compound is a small molecule inhibitor, polypeptide, antibody or antigen-binding fragment.

In one aspect, the invention provides use of a CCDC112 inhibitor in any one of the following:
a) antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
b) preparation of a product for antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
c) prevention or treatment of a tumor;
d) preparation of a drug for preventing or treating a tumor;
e) regulation of immune response against a tumor;
f) preparation of a drug for regulating immune response against a tumor;
g) inhibiting tumor cell growth in vivo and in vitro; and
h) preparation of a reagent for inhibiting tumor cell growth in vivo and in vitro.

The inhibitor can inhibit or block the binding of CCDC112 to the receptor LILRB2, LTBR, LILRB4 or CD30 of CCDC112; preferably, the inhibitor is a small molecule inhibitor, polypeptide, antibody or antigen-binding fragment.

In one aspect, the invention further provides a method for preventing or treating a tumor, including:
contacting immune cells or tumor cells of a subject with an effective dose of CCDC112 antibody, wherein the antibody can inhibit the binding of CCDC112 to a receptor LILRB2, LTBR, LILRB4, or CD30;
preferably, the subject has received or is receiving or will receive an additional anticancer therapy.

More preferably, the additional anticancer therapy includes surgery, radiotherapy, chemotherapy, immunotherapy or hormone therapy.

In one aspect, the invention further provides a companion diagnostic method for tumor immunotherapy, including: detecting the expression level or function of CCDC112 protein in tumor cells of a subject before/after immunotherapy.

In one aspect, the invention further provides an isolated antibody or an antigen-binding fragment thereof, wherein
a) the antibody specifically binds to the CCDC112 protein with high affinity; and
b) the antibody can inhibit or block the interaction between CCDC112 and any one of LILRB2, LTBR, LILRB4, and CD30.

In some embodiments, the antibody additionally has at least one of the following properties of:
i. reducing the ratio of immunosuppressive cells, MDSCs;
ii. reducing the ratio of immunosuppressive cells, Treg cells; and
iii. enhancing the killing effect of immune cells (such as PBMCs) on tumor cells.

In some embodiments, the antibody or the antigen-binding fragment thereof comprises three CDRs in a heavy-chain variable region with the amino acid sequence set forth in SEQ ID NO: 13 and three CDRs in a light-chain variable region with the amino acid sequence set forth in SEQ ID NO: 14; or a variant with one or more CDRs each having no more than two amino acid changes relative to the above six CDRs.

In some embodiments, the antibody comprises the following CDR sequences:
i. HCDR1 having an amino acid sequence as set forth in SEQ ID NO: 15, 21, 26, 38 or 30;
ii. HCDR2 having an amino acid sequence as set forth in SEQ ID NO: 16, 22, 27, 29 or 31;
iii. HCDR3 having an amino acid sequence as set forth in SEQ ID NO: 17, 23 or 32;
iv. LCDR1 having an amino acid sequence as set forth in SEQ ID NO: 18, 24 or 33;
v. LCDR2 having an amino acid sequence as set forth in SEQ ID NO: 19, 25 or 34; and
vi. LCDR3 having an amino acid sequence as set forth in SEQ ID NO: 20 or 35; or
   a variant with one or more CDRs each having no more than two amino acid changes relative to the above six CDRs i-vi.

In some specific embodiments, the CDR sequences are as follows: the heavy-chain variable region comprising the sequences of HCDR1, HCDR2 and HCDR3 defined according to IMGT; and the light-chain variable region comprising the sequences of LCDR1, LCDR2 and LCDR3 defined according to IMGT, wherein
i. the HCDR1 has the amino acid sequence as set forth in SEQ ID NO: 15;
ii. the HCDR2 has the amino acid sequence as set forth in SEQ ID NO: 16;
iii. the HCDR3 has the amino acid sequence as set forth in SEQ ID NO: 17;
iv. the LCDR1 has the amino acid sequence as set forth in SEQ ID NO: 18;
v. the LCDR2 has the amino acid sequence as set forth in SEQ ID NO: 19; and
vi. the LCDR3 has the amino acid sequence as set forth in SEQ ID NO: 20; or
   a variant with one or more CDRs each having no more than two amino acid changes relative to the above six CDRs i-vi.

Preferably, the binding KD of the antibody or the antigen-binding fragment thereof to CCDC112 satisfies that KD <2×10⁻⁸.

More preferably, KD <2×10⁻⁸, <1×10⁻⁸, <9×10⁻⁹, <8×10⁻⁹, <7×10⁻⁹, <6×10⁻⁹, <5× 10⁻⁹, <4× 10⁻⁹, <3×10⁻⁹, <2× 10⁻⁹, <1×10⁻⁹ or <1×10⁻¹⁰.

In some embodiments, the antibody or the antigen-binding fragment comprises a heavy-chain variable region and a light-chain variable region, wherein
a) the amino acid sequence of the heavy-chain variable region comprises an amino acid sequence selected from a group consisting of SEQ ID NO: 13 or has at least 70%, 80%, 90%, 95% or 99% sequence identity to a sequence in the group; and
b) the amino acid sequence of the light-chain variable region comprises an amino acid sequence selected from a group consisting of SEQ ID NO: 14 or has at least 70%, 80%, 90%, 95% or 99% sequence identity to a sequence in the group.

In some embodiments, the antibody further comprises a conjugation moiety linked to a polypeptide, the conjugation moiety is one or more of a radionuclide, a drug, a toxin, a cytokine, an enzyme, a fluorescein, a carrier protein, a lipid, and a biotin, wherein the polypeptide or antibody and the conjugation moiety can be selectively linked through a linker.

Preferably, the linker is a peptide or a polypeptide.

In some preferred embodiments, the antibody is selected from a monoclonal antibody, a polyclonal antibody, an antiserum, a chimeric antibody, a humanized antibody and a human antibody; more preferably, the antibody is selected from a multispecific antibody, a single-chain Fv (scFv), a single-chain antibody, an anti-idiotypic (anti-Id) antibody, a diabody, a minibody, a nanobody, a single-domain antibody, a Fab fragment, a F(ab') fragment, a disulfide-linked bispecific Fv (sdFv) and intrabody.

In one aspect, the invention further provides an isolated polynucleotide, wherein the polynucleotide encodes the above-mentioned antibody or antigen-binding fragment.

In one aspect, the invention further provides a recombinant vector, comprising the above-mentioned polynucleotide, and an optional regulatory sequence; preferably, the recombinant vector is a cloning vector or an expression vector; more preferably, the regulatory sequence is selected from a leader sequence, a polyadenylation sequence, a propeptide sequence, a promoter, a signal sequence, a transcription terminator, or any combination thereof.

In one aspect, the invention further provides a host cell, wherein the host cell comprises the above-mentioned recombinant vector; preferably, the host cell is a prokaryotic cell or a eukaryotic cell.

In one aspect, the invention further provides a pharmaceutical composition, wherein the pharmaceutical composition comprises one or more of the above-mentioned antibodies or antigen-binding fragments, polynucleotides, recombinant vectors, host cells and pharmaceutical compositions;
preferably, the composition further comprises a pharmaceutically acceptable carrier or adjuvant.

In one aspect, the invention also provides a kit, wherein the kit comprises one or more of the above-mentioned antibodies or the antigen-binding fragments, polynucleotides, recombinant vectors, host cells, and pharmaceutical compositions and contained in a suitable container.

In one aspect, the invention further provides use of the above-mentioned antibody or antigen-binding fragment, polynucleotide, recombinant vector, host cell and pharmaceutical composition in any one of the following:
a) agonizing or antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
b) preparation of a product for agonizing or antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
c) prevention or treatment of a tumor;
d) preparation of a drug for preventing or treating a tumor;
e) regulation of immune response against a tumor;
f) preparation of a drug for regulating immune response against a tumor;
g) inhibiting tumor cell growth in vivo and in vitro; and
h) preparation of a reagent for inhibiting tumor cell growth in vivo and in vitro.

In one aspect, the invention further provides a method for preventing or treating a tumor, wherein the method includes:
contacting immune cells and tumor cells from a subject with an effective dose of the above-mentioned antibody or antigen-binding fragment, polynucleotide, recombinant vector, host cell, and pharmaceutical composition;
optionally, before contacting the immune cells and/or tumor cells from the subject with an effective dose of the compound, detecting the expression of CCDC112 in the tumor cells;
preferably, the immune cells are lymphocytes, more preferably, T lymphocytes;
more preferably, the subject has received or is receiving or will receive an additional anticancer therapy;

Further preferably, the additional anticancer therapy includes surgery, radiotherapy, chemotherapy, immunotherapy or hormone therapy.

In one aspect, the invention further provides a method for detecting the existence of CCDC112 in a biological sample in vivo and in vitro, including: contacting the biological sample with an effective dose of the above-mentioned antibody or antigen-binding fragment, polynucleotide, recombinant vector, host cell and pharmaceutical composition.

In one aspect, the invention further provides a method for inhibiting tumor cell growth, including the following steps:
A) analyzing the expression of CCDC112 in tumor cells; and
B) contacting the tumor cells with an antibody that can identify CCDC112;
C) contacting T lymphocytes, the antibody and the tumor cells with each other.

In some embodiments, the binding KD of the antibody to CCDC112 satisfies that KD <2×10⁻⁸; preferably, KD <2×10⁻⁸, <1×10⁻⁸, <9×10⁻⁹, <8×10⁻⁹, <7×10⁻⁹, <6×10⁻⁹, <5×10⁻⁹, <4×10⁻⁹, <3×10⁻⁹, <2×10⁻⁹, <1×10⁻⁹, or <1×10⁻¹⁰.

In other embodiments, the antibody inhibits or blocks the binding of CCDC112 to a receptor LILRB2, LTBR, LILRB4 or CD30.

In some embodiments, the above-mentioned tumor is selected from colorectal cancer, lung cancer, breast cancer, melanoma, lymphoma, liver cancer, head and neck cancer, gastric cancer, kidney cancer, bladder cancer, prostate cancer, testicular cancer, endometrial cancer, breast cancer, and ovarian cancer. Compared with the prior art, the invention at least has the following beneficial technical effects:
1) The invention defines the widespread expression of CCDC112 in tumor cells, which can be used as a biomarker for tumor diagnosis.
2) The invention discovered novel CCDC112 binding molecules, LILBR2, LIRB4, LTBR and CD30. In view of the important role of the above molecules in immune regulation and tumorigenic development, CCDC112 molecules can take effect in tumor development by increasing MDSCs and Treg cells.
3) It is found in the invention that the antibody molecule for CCDC112 can block the binding of LILBR2, LIRB4, LTBR and CD30 to CCDC112.
4) The antibody molecules discovered by the invention can promote the killing effect of PBMCs on tumor cells expressing CCDC112.
5) The invention provides a novel targeting approach for the treatment of tumors expressing CCDC112.

The foregoing are general descriptions which may include simplifications, generalizations and omissions of details where necessary. Accordingly, those skilled in the art will understand that this general description is illustrative only and is not intended to be limiting in any way. Other aspects, features and advantages of the methods, compositions and/or devices described herein and/or other subject matters will become apparent from the teachings presented herein. General descriptions are provided to briefly introduce some selected concepts and these general descriptions will be further described below in the Detailed Description. The above general descriptions are not intended to identify key features or essential features of subject matters claimed, nor are they intended to be used as an aid in determining the scope of the subject matters claimed. Furthermore, the contents of all references, patents, and published patent applications cited throughout the invention are hereby incorporated by reference in their entirety.

### BRIEF DESCRIPTION OF THE SEVERAL VIEWS OF THE DRAWINGS

In order to more clearly illustrate the technical solutions in the specific embodiments of the invention or in the prior art, drawings to be used for description of embodiments or the prior art will be introduced briefly hereinafter. Obviously, drawings referred to in the following description are some embodiments of the invention, and those skilled in the art may also conclude other drawings based on these drawings without paying creative efforts.
Fig. 1 shows the killing effect of PBMCs on co-cultured cells. Tumor cells (MCF7 human breast cancer cells (left) and colorectal cancer cells HCT116 expressing CCDC112 (right)) were co-cultured with activated human peripheral blood mononuclear cells (PBMCs). The results show that overexpression of CCDC112 inhibits the killing effect of PBMCs on tumor cells, while knocking out CCDC112 gene increases the killing effect of PBMCs on tumor cells.
Fig. 2 shows the effect of tumor cells on the ratios of MDSC (A) and Treg (B) in co-cultured PBMCs. Compared with normal HCT116 cells, HCT116 cells expressing CCDC112 were co-cultured with human PBMCs, which increased the ratios of MDSCs (HLA-DR-CD11b+CD33+) and Treg cells (CD4+CD25+Foxp3+), while CCDC112-knockout tumor cells decreased the ratios of MDSCs and Treg cells.
Fig. 3 shows the concentration gradient effect of CCDC112 binding to LILRB2 and LILRB4.
Fig. 4 shows the concentration gradient effect of CCDC112 binding to LTBR at different pH values.
Fig. 5 shows the concentration gradient effect of CCDC112 binding to CD30 at different pH values.
Fig. 6 shows expression levels of CCDC112 in different tumor cells. RL lymphoma cells have negative expression; A375 melanoma and RAJI lymphoma have low expression; MCF7 breast cancer, A549 lung cancer, HCT116, LOVO, SW1116 colorectal cancer have positive expression; SW48 colorectal cancer has strong positive expression.
Fig. 7 shows concentration-dependent effects of CCDC112 antibody binding to full-length CCDC112 protein and CCDC112 (73-227) fragment detected by ELISA.
Fig. 8 shows the results of flow cytometry, indicating that the antibody binds to HCT116 tumor cells instead of CCDC112-knockout HCT116 tumor cells.
Fig. 9 shows inhibitory effects of different concentrations of CCDC112 antibodies on the binding of CCDC112 to its receptor LILRB2/LTBR.
Fig. 10 shows the results of flow cytometry, indicating that the CCDC112 antibody inhibits the binding of tumor cells to the CCDC112 receptor (LILRB2).
Fig. 11 shows the results of flow cytometry, indicating that the CCDC112 antibody inhibits the binding of tumor cells to the CCDC112 receptor (LTBR).
Fig. 12 shows determination of affinity constant of S-78-02 to CCDC112 by bio-layer interferometry.
Fig. 13 shows that the antibody can promote PBMC to kill different tumor cells.
Fig. 14 shows that the CCDC112 antibody can promote phagocytosis of tumor cells by macrophages.
Fig. 15 shows inhibitory effect of the CCDC112 antibody on SW48 colorectal cancer in PBMC humanized mice.
Fig. 16 shows inhibitory effect of the CCDC112 antibody on HCT116 colorectal cancer in PBMC humanized mice.
Fig. 17 shows inhibitory effect of the CCDC112 antibody on A549 lung cancer in PBMC humanized mice.
Fig. 18 shows that the CCDC112 antibody has no significant inhibitory effect on MCF7 lymphoma cells in PBMC humanized mice.
Fig. 19 shows that the CCDC112 antibody has no significant inhibitory effect on RL lymphoma cells in PBMC humanized mice.

### DETAILED DESCRIPTION OF THE INVENTION

While the invention may be embodied in many different forms, disclosed herein are specific illustrative embodiments thereof that demonstrate the principles of the invention. It should be emphasized that the invention is not limited to the particular embodiments illustrated. Furthermore, any section headings used herein are for organizational purposes only and are not to be construed as limiting the described subject matter.

The following terms or definitions are provided only to help the understanding of the invention. These definitions should not be construed as having a scope less than that understood by those skilled in the art.

Unless otherwise defined hereinafter, all technical and scientific terms used in the detailed description of the invention have the same meanings as commonly understood by those skilled in the art. Although it is believed that the following terms are well understood by those skilled in the art, the following definitions are still set forth to better explain the invention.

The terms " including", " comprising ", "having", "containing" or "involving" are inclusive or open-ended and do not exclude other unrecited elements or method steps. The term "consisting of" is considered as a preferred embodiment of the term "comprising". If in the following, a certain group is defined as including at least a certain number of embodiments, this is also should be understood as revealing a group which preferably consists only of these embodiments.

The use of an indefinite or definite article when referring to a noun in the singular, e.g., "a" or "an", "the", includes the plural form of that noun.

In addition, the terms "first", "second", "third", (a), (b), (c) and the like in the description and claims are used to distinguish similar elements and are not necessary to describe the order or time order. It should be understood that the terms so used are interchangeable under appropriate circumstances and that the embodiments described herein are capable of operation in other orders than described or illustrated herein.

The term "and/or" is considered as a specific disclosure that each of two specified features or components is with or without the other. Thus, the term "and/or" as used herein in phrases such as "A and/or B" is intended to include both A and B; A or B; A (alone); and B (alone). Likewise, the term "and/or" as used in phrases such as "A, B, and/or C" is intended to encompass each of the following: A, B, and C; A, B, or C; A, or C; A or B; B or C; A and C; A and B; B and C; A (alone); B (alone); and C (alone).

The terms "for example" and "that is" are used by way of example only, not intended to be limiting, and should not be construed as referring to only those items explicitly recited in the description.

The terms "or more", "at least", "more than" etc., for example, "at least one", should be construed as including but not limited to at least 1, 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or 200, 300, 400, 500, 600, 700, 800, 900, 1000, 2000, 3000, 4000, 5000 or more than the stated value. Any greater numbers or fractions therebetween are also included.

Conversely, the term "not more than" includes every value that is less than the stated value. For example, "not more than 100 nucleotides" means including 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1 and 0 nucleotide(s). Any smaller numbers or fractions therebetween are also included.

The terms "a plurality of", "at least two", "two or more", "at least a second", etc. shall be construed as including but not limited to at least 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or 200, 300, 400, 500, 600, 700, 800, 900, 1000, 2000, 3000, 4000, 5000 or more. Any greater numbers or fractions therebetween are also included.

The terms "about" and "approximately" represent the range of accuracy that can be understood by those skilled in the art and still guarantee the technical effect of the mentioned feature. The term generally means a deviation of ±10%, preferably ±5%, from the indicated value.

As stated herein, unless otherwise indicated, any concentration range, percentage range, ratio range, or integer range should be understood to include the value of any integer within the stated range and, where appropriate, fractions thereof (e.g., one-tenth and one-hundredth of an integer).

As used herein, the term "antibody" (Ab) includes, but is not limited to, a glycoprotein immunoglobulin that specifically binds to an antigen. Generally, an antibody may comprise at least two heavy (H) chains and two light (L) chains linked by disulfide bonds, or an antigen-binding molecule thereof. Each heavy chain is comprised of a heavy-chain variable region (VH) and a heavy-chain constant region. The heavy-chain constant region comprises three constant domains, i.e., CH1, CH2 and CH3. Each light chain is comprised of a light-chain variable region (VL) and a light-chain constant region. The light-chain constant region comprises one constant domain (CL). The VH and VL can be further subdivided into hypervariable regions called complementarity determining regions (CDRs), interspersed with more conserved regions called framework regions (FRs). The VH and the VL each comprise three CDRs and four FRs, arranged from amino-terminus to carboxy-terminus in the following order: FR1, CDR1, FR2, CDR2, FR3, CDR3, and FR4. The variable regions of the heavy and light chains contain binding domains that interact with the antigen. The constant regions of Ab can mediate the binding of the immunoglobulin to host tissues or factors and include various cells of the immune system (e.g., effector cells) and the first component (Clq) of the classical complement system.

The VH and the VL each comprise a "framework" region interspersed with three hypervariable regions (also called "complementarity determining regions" or "CDRs"). A "complementarity determining region" or "CDR" or "hypervariable region" (used interchangeably herein with a hypervariable region "HVR") is a region that is hypervariable in sequence in an antibody variable domain and is a structurally defined loop ("hypervariable loop") and/or contains an antigen contact residue ("antigen contact site"). The CDRs are primarily responsible for binding to antigenic epitopes. The CDRs of the heavy and light chains are commonly referred to as CDR1, CDR2 and CDR3, numbered sequentially starting from the N-terminus. The CDRs located in the VH of the antibody are called HCDR1, HCDR2 and HCDR3, and the CDRs located in the VL of the antibody are called LCDR1, LCDR2 and LCDR3. In a given VL or VH amino acid sequence, the precise amino acid sequence boundaries of each CDR can be determined using any one or combination of a number of well-known antibody CDR assignment systems. The assignment systems include, for example, Chothia based on the three-dimensional structure of the antibody and the topology of the CDR loops (Chothia et al. (1989) Nature 342:877-883, Al-Lazikani et al, "Standard conformations for the canonical structures of immunoglobulins", Journal of Molecular Biology, 273,927-948(1997)), Kabat based on antibody sequence variability (Kabat et al, Sequence of Proteins of Immunological Interest, 4th Version, U.S. Department of Health and Human Services, National Institutes of Health(1987)), AbM (University of Bath), Contact (University College London), international ImMunoGeneTics database (IMGT), and North based on affinity propagation clustering using a large number of crystal structures for CDR definition.

However, it should be noted that the boundaries of the CDRs of the variable region of the same antibody obtained based on different assignment systems may be different. That is, the CDR sequences of the variable region of the same antibody defined under different assignment systems are different. For example, the residue ranges defined by different assignment systems for CDR regions numbered by Kabat and Chothia are shown in Table A below.

**Table A. CDR residue ranges defined by different assignment systems**

| Loop | Kabat CDR | AbM | Chothia | Contact | IMGT |
|---|---|---|---|---|---|
| L1 | L24-L34 | L24-L34 | L24-L34 | L30-L36 | L27-L32 |
| L2 | L50-L56 | L50-L56 | L50-L56 | L46-L55 | L50-L52 |
| L3 | L89-L97 | L89-L97 | L89-L97 | L89-L96 | L89-L96 |
| H1 | H31-H35b Kabal No. | H26-H35b | H26-H32...34 | H30-H35b | H26-H35b |
| H1 | H31-H35 Chothia No. | H26-H35 | H26-H32 | H30-H35 | H26-H35 |
| H2 | H50-H65 | H50-H58 | H52-H56 | H47-H58 | H51-H57 |
| H3 | H95-H102 | H95-H102 | H95-H102 | H93-H101 | H93 H102 |

Thus, where reference is made to defining an antibody with a particular CDR sequence as defined in the invention, the scope of the antibody also covers antibodies of which the variable region sequences comprise the particular CDR sequences, but the application of a different protocol (e.g., different assignment system rules or combinations) causes the claimed CDR boundary to be different from the specific CDR boundary defined in the invention.

The CDRs of the antibody of the invention can be manually assessed to determine boundaries according to any protocol in the art or a combination thereof. Unless otherwise stated, in the invention, the term "CDR" or "CDR sequence" covers a CDR sequence determined in any of the above ways.

The term "antibody" can include, for example, monoclonal antibodies, recombinantly produced antibodies, monospecific antibodies, multispecific antibodies (including bispecific antibodies), human antibodies, engineered antibodies, humanized antibodies, chimeric antibodies, immunoglobulins, synthetic antibodies, tetrameric antibody comprising two heavy chain molecules and two light chain molecules, antibody light-chain monomers, antibody heavy-chain monomers, antibody light-chain dimers, antibody heavy-chain dimers, antibody light chain-antibody heavy chain pairs, intrabodies, antibody fusions (sometimes referred to herein as "antibody conjugates"), heteroconjugated antibodies, single-domain antibodies, monovalent antibodies, single-chain antibodies or single-chain Fv (scFv) fragments, camelized antibodies, affibodies, Fab fragments, F(ab')2 fragments, disulfide-linked Fv (sdFv) fragments, anti-idiotypic (anti-Id) antibodies (including, for example, anti-anti-Id antibodies), micro antibodies, domain antibodies, synthetic antibodies (sometimes referred to herein as "antibody mimetics"), and antigen-binding fragments of any of the above.

The term "humanized antibody" is intended to mean an antibody obtained by grafting CDR sequences derived from the germline of another mammalian species, such as mice, onto human framework sequences. Additional framework region modifications can be made in the human framework sequences.

As used herein, "antigen-binding molecule", "antigen-binding fragment" or "antibody fragment" refers to any molecule comprising an antigen-binding fragment (e.g., CDR) of an antibody from which the molecule is derived. The "antigen-binding molecule" may include complementarity determining regions (CDRs). Examples of antibody fragments include, but are not limited to, Fab, Fab', F(ab')2 and Fv fragments, dAbs, linear antibodies, scFv antibodies and multispecific antibodies formed from antigen-binding molecules. In some embodiments, the antigen-binding molecule binds to CCDC112 protein. In some embodiments, the antigen-binding molecule has neutralizing activity and can inhibit the binding of CCDC112 to the receptor LILRB2, LTBR, LILRB4, or CD30.

As used herein, the term "antigen" refers to any molecule that elicits an immune response or is capable of being bound by an antibody or antigen-binding molecule. The immune response may involve antibody production or activation of specific immunocompetent cells, or both. Those skilled in the art will readily appreciate that any macromolecule, including virtually any protein or peptide, can serve as an antigen. Antigens may be expressed endogenously, i.e., from genomic DNA, or may be expressed recombinantly. An antigen may be specific for certain tissues, such as cancer cells, or it may be expressed broadly. In addition, fragments of large molecules can function as antigens. In some embodiments, the antigen is a CCDC112 protein antigen.

As used herein, in some embodiments, the antigen binding molecule, scFv, antibody or fragment thereof directly blocks the binding site on the ligand or alters the binding ability of the ligand through indirect means such as structural of the ligand or energetic changes. In some embodiments, the antigen-binding molecule, scFv, antibody or fragment thereof prevents the biological function of the protein to which it binds.

As used herein, the terms "peptide", "polypeptide" and "protein" are used interchangeably and refer to a compound comprising amino acid residues covalently linked by peptide bonds. A protein or peptide comprises at least two amino acids and there is no limit to the maximum number of amino acids that can be contained in the sequence of the protein or peptide. Polypeptides include any peptide or protein comprising two or more amino acids linked to each other by peptide bonds. As used herein, the term refers to both short chains (also commonly referred to in the art as, e.g., peptides, oligopeptides, and oligomers) and long chains (commonly referred to in the art as proteins, which come in many types). The term "polypeptide" includes, for example, biologically active fragments, substantially homologous polypeptides, oligopeptides, homodimers, heterodimers, variants of polypeptides, modified polypeptides, derivatives, analogs, fusion proteins, etc. Polypeptides include natural peptides, recombinant peptides, synthetic peptides or combinations thereof.

As used herein, the term "specifically bind" or "specifically bind to" refers to a non-random binding reaction between two molecules, such as between an antibody and an antigen.

As used herein, the ability to "inhibit binding", "block binding" or "compete for the same epitope" refers to the ability of an antibody to inhibit the binding of two molecules to any detectable extent. In some embodiments, an antibody that blocks binding between two molecules inhibits the binding interaction between the two molecules by at least 50%. In some embodiments, the inhibition may be greater than 60%, greater than 70%, greater than 80%, or greater than 90%.

As used herein, the term "Ka" is intended to denote the association rate of a particular antibody-antigen interaction, while the term "Kd" as used herein is intended to denote the dissociation rate of a particular antibody-antigen interaction. As used herein, the term "KD" or "KD value" is intended to mean the dissociation constant for a particular antibody-antigen interaction, which is obtained from the ratio of Kd to Ka (i.e., Kd/Ka) and expressed as a molar concentration (M). The KD value of an antibody can be determined using methods well established in the art.

As used herein, the term "high-affinity" antibody refers to an antibody with a KD value of 1×10⁻⁷ M or lower, more preferably 5×10⁻⁸ M or lower, even more preferably 1×10⁻⁸ M or lower, even more preferably 5×10⁻⁹ M or lower, and even more preferably 1×10⁻⁹ M or lower against a target antigen.

As used herein, the term "epitope" refers to the portion of an antigen to which an immunoglobulin or antibody specifically binds. An "epitope" is also referred to as an "antigenic determinant". Epitopes or antigenic determinants usually consist of chemically active surface groups of molecules such as amino acids, carbohydrates or sugar side chains, and usually have a specific three-dimensional structure and specific charge characteristics. For example, an epitope typically comprises at least 3, 4, 5, 6, 7, 8, 9, 10, 11, 12, 13, 14, or 15 consecutive or discontiguous amino acids in a unique three-dimensional conformation, and it may be a "linear epitope" or "conformational epitope". Please refer to, for example, Epitope Mapping Protocols in Methods in Molecular Biology, Vol. 66, G.E. Morris, Ed. (1996). In a linear epitope, all interaction sites between a protein and an interacting molecule (such as an antibody) occur linearly along the primary amino acid sequence of the protein. In a conformational epitope, the interaction sites span amino acid residues that are separated from each other in the protein. Antibodies can be screened depending on their competition for binding to the same epitope as detected by conventional techniques known to those skilled in the art. For example, competition or cross-competition studies can be performed to obtain antibodies that compete or cross-compete with each other for binding to an antigen (e.g., CLDN18.2). A high-throughput method for obtaining antibodies binding to the same epitope, based on their cross-competition, is described in International Patent Application WO 03/048731.

The term "nucleic acid" or "nucleic acid sequence" in the invention refers to any molecule, preferably a polymeric molecule, comprising ribonucleic acid, deoxyribonucleic acid or analog units thereof. The nucleic acid can be single-stranded or double-stranded. A single-stranded nucleic acid may be the nucleic acid of one strand of denatured double-stranded DNA. Alternatively, a single-stranded nucleic acid may be a single-stranded nucleic acid that is not derived from any double-stranded DNA.

The term "complementary" as used herein refers to the hydrogen bonding base pairing between the nucleotide bases G, A, T, C and U such that when two given polynucleotides or polynucleotide sequences anneal to each other, in DNA, A is paired with T, G is paired with C, and in RNA, G is paired with C, and A is paired with U.

As used herein, the term "cancer" refers to a large group of various diseases characterized by the uncontrolled growth of abnormal cells in the body. Unregulated cell division and growth leads to the formation of malignant tumors that invade adjacent tissues and can metastasize to remote parts of the body through the lymphatic system or bloodstream. The term "cancer" or "cancerous tissue" may include tumors. Examples of the cancer or cancerous tissue are bone cancer, pancreatic cancer, skin cancer, head or neck cancer, skin or eye malignant melanoma, uterine cancer, ovarian cancer, rectal cancer, anal region cancer, gastrointestinal cancer, testicular cancer, uterine cancer, fallopian tube cancer, endometrial cancer, cervical cancer, vaginal cancer, vulvar cancer, Hodgkin's disease, non-Hodgkin's lymphoma, esophagus cancer, small intestine cancer, endocrine system cancer, thyroid cancer, parathyroid cancer, adrenal gland cancer, soft tissue sarcoma, tract cancer, penile cancer, chronic or acute leukemia (including acute myeloid leukemia, chronic myeloid leukemia, acute lymphoblastic leukemia, chronic lymphocytic leukemia), childhood solid tumors, lymphocytic lymphoma, bladder cancer, kidney or ureter cancer, renal pelvis cancer, neoplasms/tumors of the central nervous system (CNS), primary CNS lymphoma, tumor angiogenesis, spinal axis tumors, brainstem gliomas, pituitary adenomas, Kaposi sarcoma, epidermis carcinomas, squamous cell carcinomas, T-cell lymphomas, environmentally induced cancers (including those induced by asbestos), and combinations of the above cancers.

As used herein, the term "CCDC112-associated cancer" refers to a tumor type in which there is abnormal expression of CCDC112, or any cancer caused by, aggravated by or otherwise associated with increased or decreased expression or activity of CCDC112. In some embodiments, the methods disclosed herein can be used for cancers selected from colorectal cancer, lung cancer, breast cancer, melanoma, lymphoma, liver cancer, head and neck cancer, stomach cancer, kidney cancer, bladder cancer, prostate cancer, testicular cancer, endometrial cancer, breast cancer, and ovarian cancer.

As used herein, an "effective dose", "effective amount" or "therapeutically effective dose" is any amount that protects a subject from onset of disease or promotes regression of disease when used alone or in combination with another therapeutic agent. Regression of the disease is evidenced by a decrease in the severity of disease symptoms, an increase in the frequency and duration of asymptomatic periods of the disease, or prevention of impairment or disability due to the affliction of the disease. The ability of a therapeutic agent to promote disease regression can be assessed using various methods known to the skilled practitioner, for example, in human subjects during clinical trials, in animal model systems for prediction of efficacy in humans, or by assaying the activity of the agent in an in vitro assay.

As used herein, an "individual" or "subject" is a mammal. Mammals include primates (e.g., humans and non-human primates such as monkeys) and rodents (e.g., mice and rats). In certain embodiments, the individual or subject is a human. A "subject" may be a "patient" (a human subject in need of treatment), may be an individual suffering from a CCDC112-associated cancer, such as colorectal cancer, a subject at risk of developing a CCDC112-associated cancer, such as colorectal cancer.

As used herein, the term "in vitro cell" refers to any cell cultured ex vivo. In particular, in vitro cells may include T cells.

As used herein, the term "pharmaceutically acceptable" means that the carrier, diluent, excipient and/or a salt thereof is chemically and/or physically compatible with the other ingredients in the formulation and physiologically compatible with the recipient.

As used herein, the term "pharmaceutically acceptable carrier and/or excipient" refers to a carrier and/or excipient that is pharmacologically and/or physiologically compatible with the subject and the active agent, is known in the art (refer to, for example, Remington's Pharmaceutical Sciences. Edited by Gennaro AR, 19th ed. Pennsylvania: Mack Publishing Company, 1995), and includes but is not limited to pH adjusters, surfactants, adjuvants and ionic strength enhancers. For example, pH adjusters include, but are not limited to, phosphate buffer. Surfactants include but are not limited to cationic, anionic or nonionic surfactants such as Tween-80. Ionic strength enhancers include but are not limited to sodium chloride.

As used herein, the term "regulation" generally includes the meaning of up-regulation or down-regulation in two different directions. In some cases, it can be understood as inhibition or enhancement, in some cases it can be understood as reduction or improvement, in some cases it can be understood as reduction or increase, etc., and its specific explanation is not limited herein, and it should be understood and interpreted according to the actual application context. Exemplarily, in some embodiments, "regulating" tumor cell growth can be understood as inhibiting or enhancing tumor cell growth.

As used herein, the terms "decrease" and "reduction" are used interchangeably and mean any change from the original. "Decrease" and "reduction" are relative terms that require a comparison between before and after measurements. "Decrease" and "reduction" include total consumption. Similarly, the terms "increase" and "enhancement" are construed oppositely.

"Therapy" or "treatment" for a subject means performing any type of intervention or procedure on a subject, or administering an active agent to a subject, in order to reverse, alleviate, ameliorate, inhibit, slow down or prevent symptoms, complications or conditions, or the onset, progression, development, severity, or recurrence of disease-related biochemical indicators. In some embodiments, "therapy" or "treatment" includes partial remission. In another embodiment, "therapy" or "treatment" includes complete remission.

Various aspects of the present disclosure are described in further detail.

### 1. CCDC112 protein and its effect in tumor inhibition

The protein CCDC112 is a "coiled-coil domain containing protein 112", which belongs to the coiled-coil domain (CCDC) family. The CCDC protein is a protein domain that exists in a variety of natural proteins. At least 200 CCDC proteins have been discovered so far. Different CCDC proteins have diverse biological functions, including participating in intercellular transmembrane signal transduction, genetic signal transcription, promoting cell proliferation and apoptosis, and regulating the invasion and metastasis of malignant tumor cells and other biological behaviors. Some known CCDC proteins are listed in the table below.

| Gene | Gene synonym | Ensembl | Gene description |
|---|---|---|---|
| CCDC102A | MGC 10992 | ENSG00000135736 | Coiled-coil domain containing 102A |
| CCDC105 | FLJ40365 | ENSG00000160994 | Coiled-coil domain containing 105 |
| CCDC106 | HSU79303 | ENSG00000173581 | Coiled-coil domain containing 106 |
| CCDC 107 | MGC31967 | ENSG00000159884 | Coiled-coil domain containing 107 |
| CCDC110 | CT52, KM-HN-1, MGC33607 | ENSG00000168491 | Coiled-coil domain containing 110 |
| CCDC112 | MGC39633 | ENSG00000164221 | Coiled-coil domain containing 112 |
| CCDC113 | DKFZp434N1418, HSPC065 | ENSG00000103021 | Coiled-coil domain containing 113 |
| CCDC114 | CILD20, FLJ32926 | ENSG00000105479 | Coiled-coil domain containing 114 |
| CCDC115 | ccp1, FLJ30131, MGC12981 | ENSG00000136710 | Coiled-coil domain containing 115 |
| CCDC116 | FLJ36046 | ENSG00000161180 | Coiled-coil domain containing 116 |
| CCDC117 | FLJ33814 | ENSG00000159873 | Coiled-coil domain containing 117 |
| CCDC12 | MGC23918 | ENSG00000160799 | Coiled-coil domain containing 12 |
| CCDC120 | JM11 | ENSG00000147144 | Coiled-coil domain containing 120 |
| CCDC121 | FLJ13646, FLJ43364 | ENSG00000176714 | Coiled-coil domain containing 121 |
| CCDC122 | FLJ31846 | ENSG00000151773 | Coiled-coil domain containing 122 |
| CCDC124 | | ENSG00000007080 | Coiled-coil domain containing 124 |
| CCDC125 | KENAE | ENSG00000183323 | Coiled-coil domain containing 125 |
| CCDC126 | FLJ23031 | ENSG00000169193 | Coiled-coil domain containing 126 |
| CCDC127 | FLJ25701 | ENSG00000164366 | Coiled-coil domain containing 127 |
| CCDC129 | FLJ38344 | ENSG00000180347 | Coiled-coil domain containing 129 |
| CCDC13 | FLJ25467 | ENSG00000244607 | Coiled-coil domain containing 13 |
| CCDC130 | MGC 10471 | ENSG00000104957 | Coiled-coil domain containing 130 |
| CCDC134 | FLJ22349 | ENSG00000100147 | Coiled-coil domain containing 134 |
| CCDC136 | DKFZP434G156, KIAA 1793 | ENSG00000128596 | Coiled-coil domain containing 136 |
| CCDC137 | MGC 16597 | ENSG00000185298 | Coiled-coil domain containing 137 |
| CCDC138 | FLJ32745 | ENSG00000163006 | Coiled-coil domain containing 138 |
| CCDC14 | DKFZp434L1050, FLJ12892 | ENSG00000175455 | Coiled-coil domain containing 14 |
| CCDC140 | FLJ32447 | ENSG00000163081 | Coiled-coil domain containing 140 |
| CCDC141 | CAMDI, FLJ39502 | ENSG00000163492 | Coiled-coil domain containing 141 |
| CCDC142 | FLJ14397 | ENSG00000135637 | Coiled-coil domain containing 142 |
| CCDC144A | FLJ43983, KIAA0565 | ENSG00000170160 | Coiled-coil domain containing 144A |
| CCDC144N L | MGC87631 | ENSG00000205212 | Coiled-coil domain containing 144 family, N-terminal like |
| CCDC146 | KIAA1505 | ENSG00000135205 | Coiled-coil domain containing 146 |
| CCDC148 | MGC125588 | ENSG00000153237 | Coiled-coil domain containing 148 |
| CCDC149 | DKFZp761B107 | ENSG00000181982 | Coiled-coil domain containing 149 |
| CCDC15 | FLJ13215 | ENSG00000149548 | Coiled-coil domain containing 15 |
| CCDC150 | FLJ39660 | ENSG00000144395 | Coiled-coil domain containing 150 |
| CCDC151 | MGC20983 | ENSG00000198003 | Coiled-coil domain containing 151 |
| CCDC152 | LOC100129792 | ENSG00000198865 | Coiled-coil domain containing 152 |
| CCDC153 | LOC283152 | ENSG00000248712 | Coiled-coil domain containing 153 |
| CCDC154 | C16orf29, LOC645811 | ENSG00000197599 | Coiled-coil domain containing 154 |
| CCDC155 | FLJ32658, KASH5 | ENSG00000161609 | Coiled-coil domain containing 155 |
| CCDC157 | | ENSG00000187860 | Coiled-coil domain containing 157 |
| CCDC158 | FLJ25770 | ENSG00000163749 | Coiled-coil domain containing 158 |
| CCDC159 | | ENSG00000183401 | Coiled-coil domain containing 159 |
| CCDC160 | | ENSG00000203952 | Coiled-coil domain containing 160 |
| CCDC163 | C1orf231, CCDC163P | ENSG00000280670 | Coiled-coil domain containing 163 |
| CCDC166 | | ENSG00000255181 | Coiled-coil domain containing 166 |
| CCDC167 | C6orf129, dJ153P14.2 | ENSG00000198937 | Coiled-coil domain containing 167 |
| CCDC168 | C13orf40, FLJ40176 | ENSG00000175820 | Coiled-coil domain containing 168 |

However, the function of CCDC112 protein has not been disclosed, and there is no report on the inhibition of tumors based on CCDC112. The inventors have confirmed through gene silencing knockout and immunology experiments that inhibiting the function or activity of CCDC112 can block the interaction between CCDC112 and receptors LILRB2, LTBR, LILRB4 or CD30, promote the killing effect of immune cells on tumor cells, so it can be used in the field of cancer treatment.

In some embodiments, the cancer is a CCDC112-associated cancer.

As used herein, the term "CCDC112-associated cancer" refers to a tumor type in which there is abnormal expression of CCDC112, or any cancer caused by, aggravated by, or otherwise associated with increased or decreased expression or activity of CCDC112.

In some embodiments, the methods disclosed herein can be used for cancers selected from colorectal cancer, lung cancer, breast cancer, melanoma, lymphoma, liver cancer, head and neck cancer, stomach cancer, kidney cancer, bladder cancer, prostate cancer, testicular cancer, endometrial cancer, and ovarian cancer.

### 2. Method for regulating tumor cells based on CCDC112

Based on the above findings of the invention, a method for regulating tumor cells in vitro or in vivo is provided. The method changes the ratio of immunosuppressive cells MDSC or Treg by changing the binding interaction between CCDC112 and any of the receptors LILRB2, LTBR, LILRB4 or CD30, thereby regulating the killing effect of PBMCs on tumor cells.

Those skilled in the art can understand that such regulation can be to regulate the cell activity of tumor cells, or the growth of tumor cells, or the migration of tumor cells, etc., and there is no limitation here. It is reasonable to expect that such regulation can be various in the art. "Regulation" includes both enhancement and inhibition. In more embodiments, this regulation is in the aspect of inhibition, i.e., inhibiting the activity, growth or migration of tumor cells, in order to achieve the purpose of removing or treating a disease focus.

In some specific inhibition embodiments, the method may include the steps:
a) for tumor cells expressing CCDC112, achieving the above purpose by inhibiting the expression, function or activity of CCDC112. Optionally, prior to step a), the method may further include detecting the expression of CCDC112 in tumor cells, so as to better implement the above method.
   Further, the method may also include step b):
b) contacting the tumor cells with immune cells. It can be understood that step b) may be prior to or subsequent to step a), which does not affect the implementation of the method.

As can be understood in the art, there are various methods for inhibiting the expression, function or activity of a specific protein. These methods can be carried out at the protein level or at the gene level, which are well known in the art.

In some embodiments, the above-mentioned inhibition method can be any of:
a. gene editing on the CCDC112 gene to destroy the integrity of the gene, thereby reducing the expression level, activity and the like of the gene.

In some preferred embodiments, the gene editing system of the invention is a CRISPR/Cas9 gene editing system.

In some more preferred embodiments, an oligomeric DNA sequence used to encode sgRNA in the CRISPR/Cas9 gene editing system is selected from SEQ ID NOs: 1-8.
b. Interfering with the expression of CCDC112 gene, by, for example, RNAi, to reduce the expression level or activity of the gene; and
c. Carrying out immunological inhibition, by, for example, inhibiting the function or activity of CCDC112 protein through an antibody.

In the other aspect, the invention can further provide regulation methods in other aspects, for example, a method for regulating the activity of LILRB2, LTBR, LILRB4 or CD30 in cells in vivo and in vitro; for example, a method for regulating the ratio of immunosuppressive cells MDSC or Treg in vivo and in vitro; for another example, a method for regulating the killing effect of PBMCs on tumor cells in vivo and in vitro. These methods all include the step of changing the function or activity of CCDC112 in tumor cells.

In some embodiments, the above regulation method is a method for inhibiting the activity of LILRB2, LTBR, LILRB4 or CD30; a method for reducing the ratio of immunosuppressive cells MDSC or Treg; a method for enhancing the killing effect of PBMCs on tumor cells. These methods respectively include the step of inhibiting the function or activity of CCDC112 in tumor cells.

### 3. Inhibitor of the invention and method for screening or identifying the inhibitor thereof

Based on the invention, it is found that the CCDC112 protein can bind to a receptor such as LILRB2, LTBR, LILRB4 or CD30, and that the expression of the CCDC112 protein in tumor cells can affect myeloid-derived suppressor cells (MDSCs), regulatory T cells (Treg) and immune cells.

The invention provides a CCDC112 inhibitor. The "inhibitor" in the invention can be an inhibitor at the protein level or an inhibitor at the gene level. Anything that can inhibit CCDC 112 gene or protein, including but not limited to the generation, transcription, activity, function, and the like of the gene, and the expression, activity, function, and the like of the protein, belongs to the scope of "inhibitor".

Exemplary,
when the inhibitor is at the protein level, it is a substance capable of binding to CCDC112 protein and also inhibiting or blocking the interaction between CCDC112 and any of LILRB2, LTBR, LILRB4 or CD30.

In some embodiments, the CCDC112 inhibitor at the protein level specifically binds to the CCDC112 protein.

In some embodiments, the CCDC112 inhibitor at the protein level specifically binds to the CCDC112 protein with high affinity.

In some embodiments, the inhibitor at the protein level is a compound that agonizes or antagonizes the interaction between CCDC112 and LILRB2, LILRB4, LTBR and CD30.

In some embodiments, the inhibitors at the protein level include, but are not limited to, small molecule inhibitors, polypeptides, antibodies, or antigen-binding fragments.

When the inhibitor is at the gene level, it is a substance that can inhibit or block the interaction between CCDC112 and any one of LILRB2, LTBR, LILRB4 or CD30 by changing the activity or function of CCDC112 gene.

In some embodiments, the CCDC112 inhibitors at the gene level include but are not limited to small molecule substances, DNA-like substances or RNA-like substances that are specific to the CCDC112 gene, such as primers, probes, sgRNA and other substances.

The invention further provides a method for screening or identifying the above-mentioned inhibitor, including the following steps:
firstly, obtaining a CCDC112 inhibitory molecule, and then analyzing the effect of candidate molecules on the binding of CCDC112 to the receptor LILRB2, LTBR, LILRB4, or CD30;
preferably, analyzing whether the candidate molecules have antagonistic activity.

In some embodiments, the method may include the following steps:
firstly, obtaining a CCDC112 inhibitory molecule, and then analyzing the effect of candidate molecules on myeloid-derived suppressor cells (MDSCs); preferably, analyzing whether the candidate molecules can reduce the content or ratio of suppressor cells (MDSCs).

In some embodiments, the method may include the following steps: firstly, obtaining a CCDC112 inhibitory molecule, and then analyzing the effect of candidate molecules on regulatory T cells (Treg); preferably, analyzing whether the candidate molecules can reduce the content or ratio of Treg.

In some embodiments, the method may include the following steps: firstly, obtaining a CCDC112 inhibitory molecule, and then analyzing the effect of candidate molecules on immune cells to kill tumor cells; preferably, analyzing whether the candidate molecules can promote immune cells to kill or inhibit tumor cells.

In some embodiments, the method may include a combination or all of the above steps.

Through the above methods, it can be expected in the art to screen and/or identify valuable CCDC112 inhibitory molecules.

### 4. Antibody of the invention and its preparation method

### Antibody of the invention

The terms "antibody against CCDC112", "anti-CCDC112 antibody", "anti-CCDC112 protein antibody", "CCDC112 protein antibody", or "CCDC112-binding antibody" are used interchangeably herein. It refers to the antibody of the invention that can bind to the CCDC112 protein with sufficient affinity, whereby the antibody can be used as a diagnostic, preventive and/or therapeutic agent targeting the CCDC112 protein.

Any antibody or antigen-binding fragment thereof having the following properties theoretically belongs to the inventive concept or scope of the invention. Specifically, the isolated antibody or antigen-binding fragment thereof can
a) specifically bind to the CCDC112 protein with high affinity; and
b) inhibit or block the interaction between CCDC112 and any one of LILRB2, LTBR, LILRB4, and CD30.

In some embodiments, the antibody additionally has at least one of the following properties of:
reducing the ratio of immunosuppressive cells, MDSCs;
reducing the ratio of immunosuppressive cells, Treg cells; and
enhancing the killing effect of immune cells (such as PBMCs) on tumor cells.

Additionally, /alternatively, the antibody may have one or more of the other above-mentioned features.

In some specific embodiments, the isolated antibody of the invention or an antigen-binding fragment thereof includes a heavy-chain variable region and a light-chain variable region, and specifically includes:
three CDRs in the heavy-chain variable region with the amino acid sequence as set in SEQ ID NO: 13 and three CDRs in the light-chain variable region with the amino acid sequence as set in SEQ ID NO: 14; or a variant with one or more CDRs each having no more than two amino acid changes relative to the above six CDRs; or a variant with one or more CDRs each having no more than two amino acid changes relative to the above six CDRs.

The amino acid change refers to an addition, deletion or substitution of amino acids. Preferably, the amino acid change is a conservative amino acid substitution.

In some embodiments, the antibody comprises CDR sequences defined differently as follows:

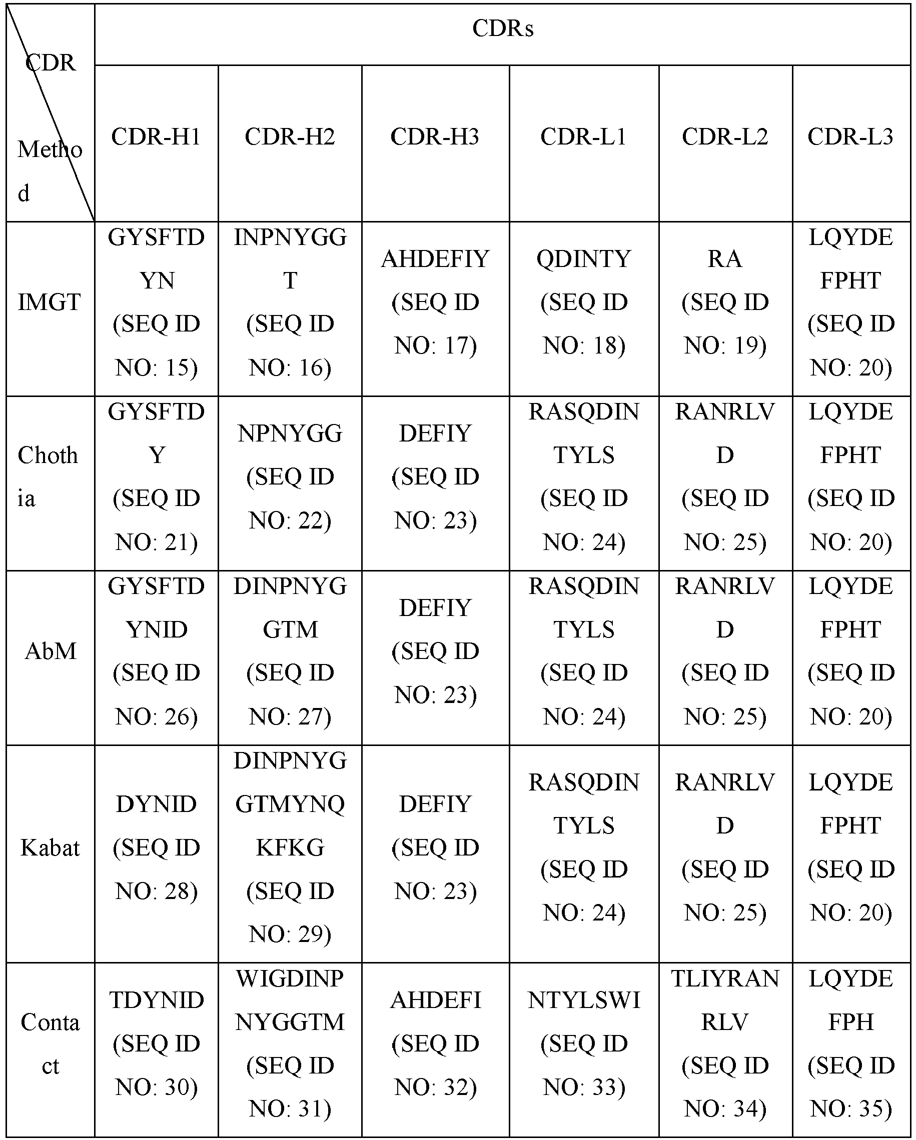

Therefore, in some embodiments, the antibody comprises the following CDR sequences:
i. HCDR1 having an amino acid sequence as set forth in SEQ ID NO: 15, 21, 26, 28 or 30;
ii. HCDR2 having an amino acid sequence as set forth in SEQ ID NO: 16, 22, 27, 29 or 31;
iii. HCDR3 having an amino acid sequence as set forth in SEQ ID NO: 17, 23 or 32;
iv. LCDR1 having an amino acid sequence as set forth in SEQ ID NO: 18, 24 or 33;
v. LCDR2 having an amino acid sequence as set forth in SEQ ID NO: 19, 25 or 34; and
vi. LCDR3 having an amino acid sequence as set forth in SEQ ID NO: 20 or 35; or
   a variant with one or more CDRs each having no more than two amino acid changes relative to the above six CDRs i-vi.

In some specific embodiments, the antibody comprises: the heavy-chain variable region comprising the sequences of HCDR1, HCDR2 and HCDR3 defined according to IMGT; and the light-chain variable region comprising the sequences of LCDR1, LCDR2 and LCDR3 defined according to IMGT, wherein
i. the HCDR1 has the amino acid sequence as set forth in SEQ ID NO: 15;
ii. the HCDR2 has the amino acid sequence as set forth in SEQ ID NO: 16;
iii. the HCDR3 has the amino acid sequence as set forth in SEQ ID NO: 17;
iv. the LCDR1 has the amino acid sequence as set forth in SEQ ID NO: 18;
v. the LCDR2 has the amino acid sequence as set forth in SEQ ID NO: 19; and
vi. the LCDR3 has the amino acid sequence as set forth in SEQ ID NO: 20; or
   a variant with one or more CDRs each having no more than two amino acid changes relative to the above six CDRs i-vi.

The amino acid change refers to an addition, deletion or substitution of amino acids. Preferably, the amino acid change is a conservative amino acid substitution.

Preferably, the binding KD of the antibody or the antigen-binding fragment thereof to CCDC112 satisfies that KD <2×10⁻⁸.

More preferably, KD <2×10⁻⁸, <1×10⁻⁸, <9×10⁻⁹, <8×10⁻⁹, <7×10⁻⁹, <6×10⁻⁹, <5× 10⁻⁹, <4×10⁻⁹, <3×10⁻⁹, <2× 10⁻⁹, <1×10⁻⁹ or <1×10⁻¹⁰.

In some embodiments, the antibody or the antigen-binding fragment comprises a heavy-chain variable region and a light-chain variable region, wherein
a) the amino acid sequence of the heavy-chain variable region includes an amino acid sequence selected from a group consisting of SEQ ID NO: 13 or has at least 70%, 80%, 90%, 95% or 99% sequence identity to a sequence in the group; and
b) the amino acid sequence of the light-chain variable region includes an amino acid sequence selected from a group consisting of SEQ ID NO: 14 or has at least 70%, 80%, 90%, 95% or 99% sequence identity to a sequence in the group.

Conservative substitution refers to the substitution of one amino acid by another amino acid within the same class, such as the substitution of one acidic amino acid by another acidic amino acid, the substitution of one basic amino acid by another basic amino acid, or the substitution of one neutral amino acid by another neutral amino acid. Exemplary substitutions are shown in the table below:

Exemplary amino acid substitutions are as follows:

| Original Residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |

| Original Residue | Exemplary substitution | Preferred substitution |
|---|---|---|
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

In preferred embodiments, the amino acid changes described in the invention occur in regions outside the CDRs (e.g., in FRs). More preferably, the amino acid changes described in the invention occur in Fc regions. In some embodiments, provided is an anti-CCDC112 protein antibody comprising an Fc domain containing one or more mutations that enhance or reduce binding of the antibody to an FcRn receptor, for example, at acidic pH as compared to neutral pH. Non-limiting examples of such Fc modifications include, for example, modifications at site 250 (e.g., E or Q), site 250 and site 428 (e.g., L or F), site 252 (e.g., L/Y/F/W or T), site 254 (e.g., S or T) and site 256 (e.g., S/R/Q/E/D or T); or modifications at site 428 and /or site 433 (e.g., H/L/R/S/P/Q or K) and/or site 434 (e.g., A, W, H, F or Y[N434A, N434W, N434H, N434F or N434Y]); or modifications at site 250 and/or 428; or modifications at site 307 or site 308 (e.g., 308F, V308F) and site 434. In one embodiment, the modifications include modifications at 428L (e.g., M428L) and 434S (e.g., N434S); modifications at 428L, 259I (e.g., V259I) and 308F (e.g., V308F); modifications at 433K (e.g., H433K) and 434 (e.g., 434Y); modifications at 252, 254 and 256 (e.g., 252Y, 254T and 256E); modifications at 250Q and 428L (e.g., T250Q and M428L); and modifications at 307 and/or 308 (e.g., 308F or 308P). In yet another embodiment, the modifications include modifications at 265A and/or 297A (e.g., N297A).

For example, the invention includes an anti-CCDC112 protein antibody comprising an Fc domain, and the Fc domain comprises a pair (group) or multiple pairs (group) of mutations selected from: mutations at 252Y, 254T and 256E (e.g., M252Y, S254T and T256E); mutations at 428L and 434S (e.g., M428L and N434S); mutations at 257I and 311I (e.g., P257I and Q311I); mutations at 257I and 434H (e.g., P257I and N434H); mutations at 376V and 434H (e.g., D376V and N434H); mutations at 307A, 380Aand 434A (e.g., T307A, E380A and N434A); and mutations at 433K and 434F(e.g., H433K and N434F). In one embodiment, the invention provides an anti-CCDC112 protein antibody comprising an Fc domain, and the Fc domain comprises a mutation at S108P in an IgG4 hinge region to promote dimer stabilization. Any possible combination of the foregoing Fc domain mutations and other mutations within the antibody variable domains disclosed herein is within the scope of the invention.

In other embodiments, the CCDC112 protein antibody provided herein can be altered to increase or decrease its degree of glycosylation. The addition or deletion of glycosylation sites for the CCDC112 protein antibody can be conveniently achieved by changing the amino acid sequence to create or remove one or more glycosylation sites. When the CCDC112 protein antibody comprises an Fc region, the type of glycosylation linked to the Fc region can be changed. In some applications, modifications to remove unwanted glycosylation sites may be useful, such as removal of fucose moieties to improve antibody-dependent cellular cytotoxicity (ADCC) function (Shield et al., (2002) JBC277: 26733). In other applications, galactosidation modifications can be made to modulate complement-dependent cytotoxicity (CDC). In some embodiments, one or more amino acid modifications can be introduced into the Fc region of the coronavirus S protein antibody provided herein to generate variants of the Fc region, so as to enhance, for example, the prevention and/or effectiveness of the coronavirus S protein antibody of the invention in treating coronavirus infection.

In some embodiments, the antibody is IgG1, IgG2, IgG3 or IgG4 antibody. Preferably, the antibody is IgG1 or IgG4 antibody. More preferably, the antibody is a human or murine IgG1 or IgG4 antibody.

In some embodiments, the antibody further comprises a conjugation moiety linked to a polypeptide, the conjugation moiety is one or more of a radionuclide, a drug, a toxin, a cytokine, an enzyme, a fluorescein, a carrier protein, a lipid, and a biotin, wherein the polypeptide or antibody and the coupling moiety can be selectively linked through a linker, and preferably the linker is a peptide or a polypeptide.

It is understood in the art that without changing the core functional region of the antibody, the antibody can be prepared into or selected from a monoclonal antibody, a polyclonal antibody, an antiserum, a chimeric antibody, a humanized antibody and a human antibody; more preferably, the antibody can be prepared into or selected from a multispecific antibody, a single-chain Fv (scFv), single-chain antibody, an anti-idiotypic (anti-Id) antibody, a diabody, a minibody, a nanobody, a single-domain antibody, a Fab fragment, a F(ab') fragment, a disulfide-linked bispecific Fv (sdFv) and intrabody.

The invention further provides an isolated polynucleotide, wherein the polynucleotide encodes the above-mentioned antibody or antigen-binding fragment.

The invention further provides a recombinant vector, comprising the above-mentioned polynucleotide, and an optional regulatory sequence.

Preferably, the recombinant vector is a cloning vector or an expression vector; more preferably, the regulatory sequence is selected from a leader sequence, a polyadenylation sequence, a propeptide sequence, a promoter, a signal sequence, a transcription terminator, or any combination thereof.

The invention further provides a host cell, comprising the above-mentioned recombinant vector. Preferably, the host cell is a prokaryotic cell or a eukaryotic cell.

The invention further provides a pharmaceutical composition, including one or more of the above-mentioned antibodies or antigen-binding fragments, polynucleotides, recombinant vectors, host cells and pharmaceutical compositions. Preferably, the composition further comprises a pharmaceutically acceptable carrier or adjuvant.

The invention also provides a kit, including one or more of the above-mentioned antibodies or antigen-binding fragments, polynucleotides, recombinant vectors, host cells, and pharmaceutical compositions and contained in a suitable container.

### Preparation method of the antibody of the invention

The anti-CCDC112 monoclonal antibody (mAb) and human sequence antibody of the invention can be produced by various techniques, including conventional monoclonal antibody methodology, such as the standard somatic cell hybridization technique (Kohler and Milstein, (1975) Nature 256:495). Any technique for the production of monoclonal antibodies may be used, such as viral or oncogenic transformation of B lymphocytes. An animal system for preparing hybridomas is a murine system. Production of hybridomas in mice is a well-established method. Immunization protocols and techniques for isolating immunized splenocytes for fusion are known in the art. In some embodiments, the invention is obtained by immunizing mice with recombinant CCDC112 protein multiple times.

### Activity assays for the antibody of the invention

The CCDC112 protein antibody provided herein can be identified, screened or characterized for their physical/chemical properties and/or biological activities by various assays known in the art. In one aspect, the antigen-binding activity of the CCDC 112 protein antibody of the invention is tested, for example, by known methods such as ELISA, Western blotting and the like. Binding to CCDC112 protein can be assayed using methods known in the art, and exemplary methods are disclosed herein. In some embodiments, the binding of the CCDC 112 protein antibody of the invention to the CCDC 112 protein is determined using biofilm layer interference, and the binding of CCDC 112 to its receptor LILRB2, LILRB4, LTBR or CD30 is further analyzed by ELISA experiments.

### 5. Pharmaceutical composition of the invention

In some embodiments, the invention provides a composition comprising any of the compounds described herein, CCDC112 protein inhibitors, antagonists, antibodies, antigenic peptides, proteins, etc. preferably the composition is a pharmaceutical composition.

In one embodiment, the composition comprises the antibody of the invention or an antigen-binding fragment thereof, or an immunoconjugate, or a bispecific molecule, and a pharmaceutically acceptable carrier.

In one embodiment, the pharmaceutical composition comprises a combination of the CCDC112 protein antibody of the invention and one or more other therapeutic agents.

In some embodiments, the pharmaceutical composition or pharmaceutical preparation of the invention comprises suitable pharmaceutical adjuvants, such as pharmaceutical carriers and pharmaceutical excipients known in the art, including buffers.

As used herein, the "pharmaceutically acceptable carrier" includes any and all solvents, dispersion media, isotonic and absorption delaying agents, and the like that are physiologically compatible. The pharmaceutical carriers suitable for use in the invention can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable excipients include starch, dextrose, lactose, sucrose, gelatin, malt, rice, flour, chalk, silica gel, sodium stearate, glyceryl monostearate, talc, sodium chloride, dried skim milk, glycerin, propylene, glycol, water, ethanol, etc. For the use of excipients and their applications, see also "Handbook of Pharmaceutical Excipients", 5th Edition, R.C. Rowe, P.J. Seskey and S.C. Owen., Pharmaceutical Press, London, Chicago. The composition, if desired, may also comprise minor amounts of wetting or emulsifying agents, or pH buffers. These compositions may take the form of solutions, suspensions, emulsions, tablets, pills, capsules, powders, sustained release formulations and the like. Oral formulations may comprise standard pharmaceutical carriers and/or excipients, such as pharmaceutical mannitol, lactose, starch, magnesium stearate, and saccharine.

A pharmaceutical preparation comprising the CCDC112 protein antibody described herein can be prepared by mixing the CCDC112 protein antibody of the invention having a desired purity with one or more optional pharmaceutical adjuvants (Remington's Pharmaceutical Sciences, 16th edition, Osol, A. Ed. (1980)), preferably in the form of a freeze-dried preparation or an aqueous solution.

The pharmaceutical compositions or preparations of the invention may also comprise more than one active ingredient as required for the particular indication being treated, preferably those with complementary activities that do not adversely affect each other. For example, it is desirable to also provide other active ingredients, including but not limited to CTLA-4 and PD-1 inhibitors and the like. The active ingredients are suitably present in combination in amounts effective for the intended use.

### 6. Preventive or therapeutic use of the invention

### Prevention or treatment method

The invention provides a method for preventing a CCDC112-associated cancer in a subject, comprising administering the inhibitor, antibody or pharmaceutical composition of the invention to the subject. Prophylactic agents can be administered prior to the manifestation of symptomatic features in order to prevent the onset of the disease, or alternatively delay the progression of the disease.

Preferably, the subject has received or is receiving or will receive an additional anticancer therapy.

More preferably, the additional anticancer therapy includes surgery, radiotherapy, chemotherapy, immunotherapy or hormone therapy.

The invention provides a method for treating a CCDC112-associated cancer in a subject, comprising administering the inhibitor, antibody or pharmaceutical composition of the invention to the subject. Therapeutic agents can be administered after the manifestation of symptoms.

Preferably, the subject has received or is receiving or will receive an additional anticancer therapy.

More preferably, the additional anticancer therapy includes surgery, radiotherapy, chemotherapy, immunotherapy or hormone therapy.

CCDC112-associated cancers include but are not limited to various cancers, such as common colorectal cancer, lung cancer, melanoma, lymphoma, liver cancer, head and neck cancer, stomach cancer, kidney cancer, bladder cancer, prostate cancer, testicular cancer, endometrial cancer, breast cancer, and ovarian cancer.

The treatment method provided by the invention can also be implemented by contacting immune cells and tumor cells from a subject with an effective dose of the above-mentioned antibody or antigen-binding fragment, polynucleotide, recombinant vector, host cell, and pharmaceutical composition. Optionally, before using an effective amount of the compound to contact the immune cells and/or tumor cells from the subject, the expression of CCDC112 in the tumor cells is detected.

Preferably, the immune cells are lymphocytes, more preferably, T lymphocytes.

### Preventive or therapeutic use

It can be understood that the use of the inhibitor, antibody or antigen-binding fragment, polynucleotide, recombinant vector, host cell and pharmaceutical composition of the invention may at least include the following preventive and therapeutic uses or the preparation of corresponding drugs in:
a) agonizing or antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
b) preparation of a product for agonizing or antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
c) prevention or treatment of a tumor;
d) preparation of a drug for preventing or treating a tumor;
e) regulation of immune response against a tumor;
f) preparation of a drug for regulating immune response against a tumor;
g) inhibiting tumor cell growth in vivo and in vitro; and
h) preparation of a reagent for inhibiting tumor cell growth in vivo and in vitro.

### 7. Diagnostic use of the invention

The term "companion diagnostic" generally refers to the provision of information about a patient's response to a particular therapeutic agent, helping to identify patient populations who could benefit from a therapeutic product, thereby improving treatment outcomes and reducing healthcare costs. In addition, the "companion diagnostic" can help identify patient populations most likely to respond to therapeutic drugs.

The invention proves that the CCDC112 protein can be used as a therapeutic target for CCDC112-associated cancers, that is, when the CCDC112 protein is expressed in tumor cells, the tumor can be effectively inhibited by introducing a CCDC112 protein inhibitor (e.g., an antibody). During the treatment of the corresponding tumor patients (including before and after treatment), the CCDC112 protein expression of tumor cells can be detected to determine the patient population most likely to respond to a therapeutic drug. Therefore, the invention provides a companion diagnostic method, which is accomplished by detecting the expression level or function of CCDC112 protein in tumor cells of a subject before/after immunotherapy.

### 8. Kit of the invention

A kit comprising an antibody or antibody composition (e.g., a human antibody, bispecific or multispecific molecule, or immunoconjugate) of the invention and instructions for use are also within the scope of the invention. The kit may further comprise at least one additional pharmaceutical agent, or one or more additional antibodies, and the like. The kit typically includes a label indicating the intended use of the kit contents. The term "label" includes any written or recorded material provided on or with the kit or otherwise accompanying the kit.

In addition, based on the diagnostic use of the invention, the kit of the invention may also include components capable of detecting the expression level or activity of CCDC112 protein in tumor cells. Therefore, in addition to the above-mentioned antibody composition, the components of the kit of the invention may also include, such as, primers, probes, etc., or other materials capable of detecting protein expression or activity, which are not limited herein.

### EXAMPLES

The experimental methods in the following examples are conventional methods unless otherwise specified. The invention will be further understood with reference to the following non-limiting experimental examples.

### Example 1: CCDC112 inhibits the killing effect of immune cells on tumor cells

Tumor cells HCT116 and MCF7, which express CCDC112 at a moderate level, were selected to knock out or overexpress CCDC112, and co-culture with PBMCs activated by CD3 and CD28. The apoptosis rate of CD45- tumor cells was analyzed by Annexin-V-labeled flow cytometry.

CCDC112 gene editing was implemented in the following steps:
1. Construction of a CRISPR/Cas9 gene editing system, i.e., a lentivirus with puromycin-resistant comprising sgRNA that specifically cuts CCDC112.
The overall process of the experiment: first synthesize the single-stranded DNA oligo of a gRNA sequence, then generate a double-stranded DNA oligo by annealing and pairing, and then directly ligate the double-stranded DNA oligo to a CRISPR/Cas9 vector through restriction sites at both ends; transfer the ligation product into prepared bacterial competent cells, send the grown monoclonal colony to the sequencing company for sequencing and identification. Correctly aligned clones are successfully constructed CRISPR/Cas9 vectors. For the target gene sequence of the target gene, gRNA oligomeric single-stranded DNA was designed and synthesized by using the gRNA sequence design principles provided in the public website according to the gene sequence (the oligo sequences are shown in SEQ ID NO: 1-8), and the corresponding double-stranded sequence was designed. The oligomeric single-stranded DNA was annealed into double-stranded gRNA oligo, and the double-stranded gRNA oligo was then inserted into the CRISPR/Cas9 vector to construct the CRISPR/Cas9 recombinant plasmid, and the CRISPR/Cas9 recombinant plasmid was then transformed into the competent cell Stbl3. The construction of the carrier includes the following specific steps:

**Table 1R Sequences of gRNA oligomeric single-stranded DNA**

| Single-stranded DNA No. | Nucleotide sequence | Sequence No. |
|---|---|---|
| sgRNAsequence. 1 | CACTTCTACATATCCCACAT | SEQ ID NO: 1 |
| sgRNAsequence.2 | GGTGGGTTTGTAAAGCCTAG | SEQ ID NO: 2 |
| sgRNAsequence.3 | CTGGTGATCATAATCATCCC | SEQ ID NO: 3 |
| sgRNAsequence.4 | TCCAGGTTGGAATAGCCCTA | SEQ ID NO: 4 |
| sgRNAsequence.5 | AATTCTAGATAGACAGGCAA | SEQ ID NO: 5 |
| sgRNAsequence.6 | TTTACAAACCCACCAAAGGT | SEQ ID NO: 6 |
| sgRNAsequence.7 | TGTTTATTATGAAAAAGCAC | SEQ ID NO: 7 |
| sgRNAsequence.8 | TAAAAAGCAGCAAAAAAGGG | SEQ ID NO: 8 |

1) Annealing of gRNA: Primers were diluted with sterile TE buffer to make the final concentration 100 µMol. 10 µL of forward and reverse primers were mixed and blown into PCR tubes for annealing, and then placed on ice for a few minutes to be direct ligated or stored in a freezer at -20°C.
2) Enzyme digestion and recovery of CRISPR/Cas9 vector: The enzyme digestion system was as follows: 5 µg of CRISPR/Cas9 vector; 10*Buffer (5 µL); 4 µL of BsmBI; ddHzO was added to reach 50 µL. Digestion was carried out at 37°C for about 30 min. During this period, a 0.8% agarose gel can be prepared, and nucleic acid electrophoresis was performed after the enzyme digestion was completed. After electrophoresis was completed, the gel strip containing the target fragment was cut out. The weight of the gel was calculated by subtracting the weight of the empty tube from the total weight measured on a balance. And the volume of the gel was calculated according to 100 mg as 100 µL. QG buffer was added in a volume three times the volume of the gel and the gel strip was placed in a 50°C water bath to completely melt the gel. During this period, the EP tube was shaken properly to speed up the dissolution of the gel. After the gel was completely melted, isopropanol was added in the same volume as the gel and mixed well. All the above liquid was transferred to the filter column and centrifuged at 13000 rpm for 30s. The liquid in the tube was discarded, and 750 µL of PE buffer was added to the column and centrifuged for 1min. The liquid in the tube was discarded and centrifuged again for 1 min. The column was transferred to a new 1.5 mL EP tube, and 50 µL of EB buffer was added to the column, centrifuged for 1 min, and the recovered vector was left in the centrifuging tube.
3) Ligation between CRISPR/Cas9 vector and primers. The ligation system was as follows: 3 µL (50 ng) of recovered vector; 1 µL (0.5 µM) of oligo primer; 1.5 µL of T4 DNA ligase buffer; 1 µL of T4 DNA ligase; ddHzO was added to reach 15 µL. The ligation system was incubated for 30 min in a 25 °C water bath.
4) Transformation: the competent cell was placed on ice and thawed naturally. All the ligation products were added into the competent cell and placed on ice for 20 min, and then heat shocked in a 42 °C water bath for 90 s. The bacterial solution was then quickly placed on ice for 2-3 min. The bacterial solution was added to 1000 µL of LB medium without antibiotics and then cultured at 37 °C and shaken at 150 rpm for 45 min. The bacterial solution was centrifuged at 3000 rpm for 2 min, about 850 µL of supernatant was discarded, and the bacterial solution at the bottom of the tube was blown and dispersed. The bacterial solution was added to a culture dish containing the corresponding resistance and spread evenly with a sterilized applicator. The culture dish was placed upside down in a 37 °C constant-temperature incubator and held overnight for cultivation.
5) Preparation of recombinant plasmid: several monoclonals were picked to undergo small-scale shaking culture.
6) Positive clones were identified by sequencing. After comparison, the sequence of the insert fragment in the recombinant clone was completely consistent with the designed oligo sequence, so the vector was constructed successfully.

2. Carrying out gene editing by using the CRISPR/Cas9 gene editing system and constructing the HCT116-CCDC112 knockout cell line
HCT116 and MCF7 cells (ATCC, VA, USA) were plated in a 24-well plate (Corning, NY, USA) at an appropriate density (the cells grew to a density of about 30-40% on the second day), and were digested and counted the next day. Firstly, the cells were infected with the quantity gradient of GFP control lentivirus (Genomeditech, Shanghai, China), the medium was changed after 48 h, and GFP fluorescence was observed under the microscope after 72 h to determine the optimal virus-to-cell infection ratio and explore the MOI value. After the MOI value was determined, the cells were re-plated. The HCT116 cells were infected with the Cas9 system lentivirus having blasticidin resistance (Genomeditech Shanghai, China) at the MOI value. The medium was changed after 48 h. A concentration gradient of blasticidin (Invivogen, CA, USA) was added to screen the cells for 10-14 days, and the cell line obtained from the final screening was HCT116 cells containing the Cas9 system. Then the HCT116 cells containing the Cas9 system were plated and counted in a 24-well plate, and the cells were infected at the MOI value with the lentivirus containing the puromycin-resistant and the sgRNA that specifically cuts CCDC112. The medium was changed after 48 h. A concentration gradient of puromycin was added to screen cells for 10-14 days to obtain the HCT116-CCDC112 knockout cell line.
3. Overexpression of the CCDC112 gene: the two ends of the nucleotide sequence encoding the CCDC112 full-length protein (see the end of Example 3: as shown in SEQ ID NO: 9) were ligated to the BamHI and KpnI cloning sites, the cDNA sequence was fully synthesized in vitro, and then digested with BamHI and KpnI, and inserted into the pcDNA3.1 vector. The clones with successful ligation were selected and verified by sequencing. HCT116 and MCF7 cells were transfected separately, and stable cells were screened with G418 to obtain HCT116-CCDC112 and MCF7-CCDC112 stable overexpression cell lines.
Nucleotide sequence of CCDC112 (SEQ ID NO: 9): 
ATGGAAAAAGATAAACACAGTCATTTCTACAACCAAAAAAGTGACTT
CAGAATTGAGCATAGTATGCTAGAAGAATTGGAAAATAAATTGATTCACAGCAG
GAAAACAGAAAGAGCAAAAATCCAGCAACAATTGGCCAAAATACATAATAATG
TAAAGAAACTTCAGCATCAATTAAAAGATGTGAAGCCTACACCTGATTTTGTTG
 
AGAAGCTCAGAGAAATGATGGAAGAAATTGAAAATGCAATTAACACTTTTAAA
GAAGAGCAGAGGTTGATATATGAAGAGCTAATTAAAGAAGAGAAGACAACTAA
TAATGAGTTGAGTGCCATATCAAGAAAAATTGACACATGGGCTTTGGGTAATTC
AGAAACAGAGAAAGCTTTCAGAGCAATCTCAAGCAAAGTTCCTGTAGACAAA
GTAACACCAAGTACTCTTCCAGAAGAGGTACTAGATTTTGAAAAATTCCTTCAG
CAAACAGGAGGGCGACAAGGTGCCTGGGATGATTATGATCACCAGAACTTTGT
AAAGGTGAGAAACAAACATAAAGGGAAGCCAACATTTATGGAAGAAGTTCTAG
AACACCTTCCTGGAAAAACACAAGATGAAGTTCAACAGCATGAAAAATGGTAT
CAAAAGTTTCTGGCTCTAGAAGAAAGAAAAAAAGAGTCAATTCAGATTTGGAA
AACTAAAAAGCAGCAAAAAAGGGAGGAAATTTTCAAGTTAAAGGAAAAGGCA
GACAACACACCTGTGCTTTTTCATAATAAACAAGAGGATAATCAAAAGCAAAA
AGAGGAACAAAGAAAGAAACAGAAATTGGCAGTTGAAGCTTGGAAGAAACA
GAAAAGTATAGAAATGTCAATGAAATGTGCTTCCCAGTTAAAAGAAGAAGAAG
AGAAAGAGAAAAAACATCAGAAAGAACGCCAGCGCCAGTTTAAGTTAAAATT
ACTACTAGAAAGTTATACCCAGCAGAAGAAAGAACAGGAAGAATTTTTGAGGC
TTGAAAAGGAGATAAGGGAAAAGGCAGAAAAGGCAGAAAAAAGGAAAAATG
CTGCTGATGAAATTTCCAGATTTCAAGAAAGAGATTTACATAAACTTGAACTGA
AAATTCTAGATAGACAGGCAAAGGAAGATGAAAAGTCACAAAAACAAAGAAG
ACTGGCAAAATTAAAAGAAAAGGTTGAAAACAATGTTAGTAGAGATCCCTCTA
GGCTTTACAAACCCACCAAAGGTTGGGAAGAACGAACCAAAAAGATAGGACC
AACAGGCTCTGGGCCACTTCTACATATCCCACATAGGGCTATTCCAACCTGGAG
ACAAGGAATACAGAGAAGAGTATGA
4. Flow cytometry proved that knocking out CCDC112 expressed by tumor cells can significantly enhance the killing effect of human peripheral blood mononuclear cells (PBMCs) on tumor cells.

CD3 and CD28 antibodies (Invitrogen, CA, USA) were mixed and diluted with PBMCs (ATCC, VA, USA) to the final concentration of 1 µg/mL to activate T cells, and the cells were cultured overnight. The next day, the PBMCs and the above-mentioned HCT116 or MCF7 cells (including cell lines knocking out or overexpressing CCDC112) were counted, and the numbers of PBMCs and HCT116 or MCF7 cells were adjusted to 1×10⁶/mL respectively, 100 µL of PBMCs and 100 µL of MCF7 cells were added to a 96-well plate (Thermo Fisher, MA, USA) and incubated in an incubator for 6 h. The 96-well plate was taken out, the cells in each well were placed in EP tubes (Axygen, CA, USA), centrifuged at 400 rcf for 5 min, and the supernatant was discarded. The cells were resuspended and washed with 500 µL of cell staining buffer (Invitrogen, CA, USA). The centrifugation and washing were repeated. The CD45-APC antibody (Invitrogen, CA, USA) was diluted with cell staining buffer at a ratio of 1 :20, 200 µL of the mixed liquid was added to each EP tube to be resuspended, and incubated at room temperature for 30 min with slow shaking. After centrifugation at 400 rcf for 5 min, the supernatant was discarded, and the cells were resuspended and washed with 1 mL of binding buffer (Meilunbio, Shanghai, China). The centrifugation and washing were repeated. Various experimental and control groups were setup, 100 µL of binding buffer, 5 µL of AnnexinV-FITC (Meilunbio, Shanghai, China) and 10 µL of PI (Meilunbio, Shanghai, China) were added respectively according to the conditions. The cells were incubated slowly at room temperature for 15 min. 400 µL of binding buffer was then added respectively and the cells were transferred to flow tubes (Falcon, NY, USA) and placed on the flow cytometer (Miltenyi, Cologne, Germany).

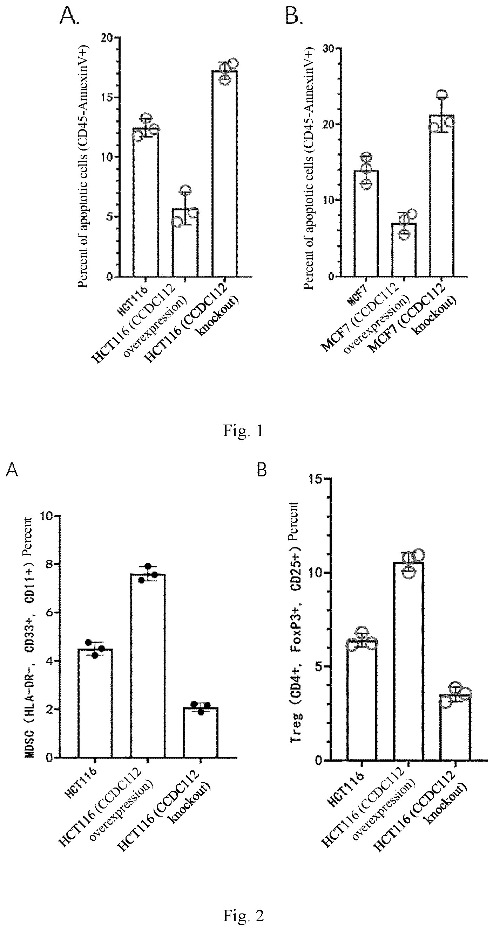

The results show that overexpression of CCDC112 inhibits the killing effect of PBMCs on tumor cells, while knocking out CCDC112 gene enhances the killing effect of PBMCs on tumor cells, as shown in Fig. 1.

### Example 2: CCDC112 increased myeloid-derived suppressor cells (MDSCs) and regulatory T cells (Treg)

In order to elucidate the effect of CCDC112 on Treg/MDSCs, firstly, cells were prepared as follows: 1.5+10E⁵ PBMCs per well were used for different experimental groups and cultured for 24h (for detecting the ratio of Treg) or 3 days (for detecting the ratio of MDSCs). Ratios and counts of phenotypic CD4+CD25+Foxp3+ and HLA-DR-CD11b+CD33+ were analyzed by flow cytometry.

As shown in the analysis results, compared with normal HCT116 cells, HCT116 cells expressing CCDC112 and human PBMCs were co-cultured, which increased the ratios of MDSCs (HLA-DR-CD11b+CD33+) and Treg cells (CD4+CD25+Foxp3+); however, CCDC112-knockout tumor cells decreased the ratios of MDSCs and Treg cells. The results are shown in Fig. 2.

### Example 3: binding of CCDC112 to receptors

The binding and binding strength of CCDC112 to LILRB2, LILRB4, LTBR and CD30 were analyzed by ELISA experiment.

Specifically, a special plate for ELISA (costar, ME, USA) was used. Firstly, different concentrations of CCDC112 recombinant proteins were dissolved in 100 µL of ELISA coating solution (Solarbio, Beijing, China) to coat the plate, and the negative control was coated with 100 µL of coating solution without protein. The plate was coated overnight at 4 °C. After washing with PBST, 100 µL of 5% BSA dissolved in PBS (VWR, PA, USA) was used for blocking in the incubator at 37 °C for 90 min. After washing with PBST, the incubation and binding was carried out in PBS solution with 1 µg/mL receptor protein (LILRB2, LILRB4, LTBR, or CD30) extracellular region protein fragments (ACRO Biosystems or Sino Biological, Beijing, China) (LTBR and CD30 were added to adjust the PBS solution to different pH values, so as to explore better binding conditions). The above-mentioned receptor protein was labeled with hFc (P-Fc) and binding was carried out in the incubator at 37 °C for 60 min. After washing with PBST, a specific anti-human Fc fragment antibody (Abcam, MA, USA) diluted in PBS was used for incubation and binding at 37 °C for 30 min. After washing with PBST, chromogenic solution (Sangon, Shanghai, China) was added at 100 µL per well for color development and reacted in an incubator for 5-30 min, and then 50 µL of stop solution (Sangon, Shanghai, China) was added, and chromogenic reading was then carried out under a microplate reader (Thermo Fisher, MA, USA) at 450nm.

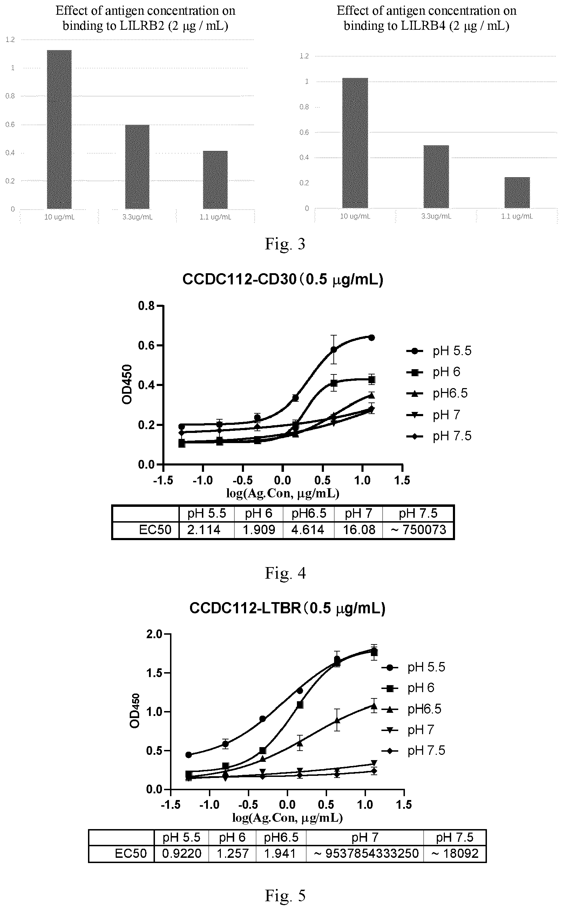

As shown in the results, CCDC112 can bind to LILRB2, LILRB4, LTBR and CD30, proving that LILRB2, LILRB4, LTBR and CD30 are all the binding receptors of CCDC112. The concentration gradient effect of CCDC112 binding to LILRB2 and LILRB4 is shown in Fig. 3. The concentration gradient effect of CCDC112 binding to LTBR at different pH values is shown in Fig. 4. The concentration gradient effect of CCDC112 binding to CD30 at different pH values is shown in Fig. 5.

### Example 4: Detection of the expression level of CCDC112 in different tumor cells.

Different tumor cell lines, including RL lymphoma cells, A375 melanoma, RAJI lymphoma, MCF7 breast cancer, A549 lung cancer, HCT116, LOVO, SW1116, SW48 colorectal cancer cells were analyzed. Specifically, the above-mentioned cultured cells were counted, 4×10⁶ cells were added into a 15mL centrifuge tube (Corning, NY, U.S.) and centrifuged at 800 rpm for 4 min, and the supernatant was then discarded. The cells were resuspended with RPMI1640 medium containing 10% calf serum (Meilunbio, Dalian, China). 1 mL of each type of cell was added into the wells of a 12-well plate (Corning, NY, USA), mixed thoroughly, and then placed in an incubator for 10 min and then taken out. The cells were transferred to a 1.5 mL EP tube (Axygen, CA, USA), centrifuged at 800 rpm for 4 min, resuspended in PBS, washed and centrifuged, and washed repeatedly. After the centrifugation was completed, the supernatant was discarded, and RIPA lysate (Beyotime, Shanghai, China) was mixed with a protease inhibitor, a phosphatase inhibitor, and PMSF (Consun, Shanghai, China) at a ratio of 1:100, and 80 µL of the mixed lysate was added to each tube of cells. Each EP tube was frozen three times and thawed three times on liquid nitrogen-ice, and centrifuged at 12000 rpm, 4 °C for 15 min after the last thaw. After the centrifugation was completed, the supernatant was taken. After the centrifugation, the supernatant: 5x loading buffer (Beyotime, Shanghai, China) was prepared into various cell samples at a ratio of 4:1, and the protein samples were then denatured in a metal bath at 100 °C for 10 min. 10% PAGE gel (Epizyme, Shanghai, China) was prepared in a gel plate (Bio-Rad, CA, USA) according to the instructions and then placed in an electrophoresis tank (Bio-Rad, CA, USA), and the power supply (Bio-Rad, CA, USA) was then turned on to let the strip run through the concentrated gel at a constant voltage of 80 volts, and run through the separating gel at a constant voltage of 120 volts. When the strips reached the bottom of the separating gel, wet transfer was carried out in a transfer tank (Bio-Rad, CA, USA) with a constant current of 350 mA for 90 min. After the transfer was completed, the membrane was sheared according to the mass of CCDC112 and GAPDH protein. Blocking with fast blocking solution (Epizyme, Shanghai, China) was carried out for ten min, and the corresponding strips were incubated with CCDC112 antibody (its preparation process is described in the example below) and GAPDH antibody (Consun, Shanghai, China) respectively, and such operations were carried out overnight at 4 °C. After washing with TBST the next day, the strips were incubated for one hour at room temperature with a specific anti-rabbit secondary antibody (Consun, Shanghai, China) diluted in 5% skimmed milk powder (Sangon, Shanghai, China) dissolved in TBS, and then washed with TBST. The strips were then placed in a mixed luminescence solution (Share-bio, Shanghai, China) for one minute and exposed under a gel imager (Bio-Rad, CA, USA).

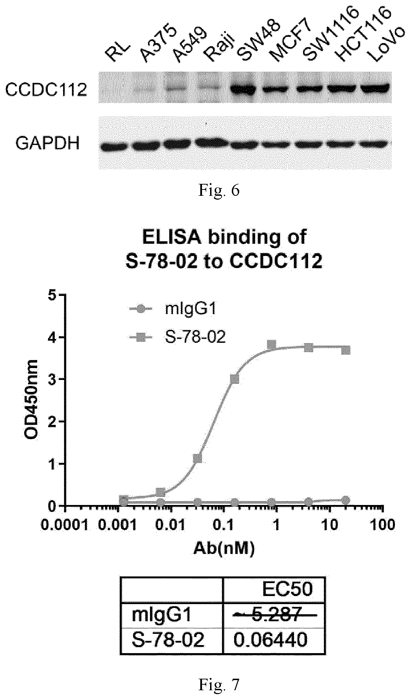

The results are shown in Fig. 6, RL lymphoma cells do not express CCDC112 (negative); A375 melanoma and RAJI lymphoma express CCDC112 at a slightly lower level; MCF7 breast cancer, lung cancer, HCT116, LOVO, and SW1116 colorectal cancer positively express CCDC112; SW48 colorectal cancer is strongly positive for CCDC112.

### Example 5: Preparation of CCDC112 monoclonal antibody

The CCDC112 protein was expressed in yeast and purified to reach a purity of 92%, and it was verified by the ELISA experiment that the CCDC112 protein has the activity of binding to receptors LILRB2, LILRB4, LTBR and CD30. CCDC112 protein (amino acid sequence shown in SEQ ID NO: 10) was used to immunize 10 mice, and the immunization was carried out many times to enhance the effect: (1) Initial immunization, antigen (50 µg/mouse) with Freund's complete adjuvant was injected subcutaneously at multiple points with an interval of 3 weeks. (2) Second immunization, the administration dose and administration route were the same as above, but Freund's incomplete adjuvant was added, and the administration interval was 3 weeks. (3) Third immunization: the administration dose was the same as above, no adjuvant was added, an intraperitoneal injection was adopted and the injection interval was 3 weeks. (4) Booster immunization, the administration dose was 50 µg, and intraperitoneal injection was adopted. Three days after the last injection, blood was collected to determine its titer and immune effect, and mice with higher titers were selected for hybridoma fusion screening. After subcloning, the binding of the monoclonal antibody to the target antigen was detected by ELISA, and the ability of different monoclonal antibodies to block the binding of CCDC 112 to the receptor was detected by ELISA.

The amino acid sequence of CCDC112 protein is shown as follows (SEQ ID NO: 10):

MEKDKHSHFYNQKSDFRIEHSMLEELENKLIHSRKTERAKIQQQLAKIH
NNVKKLQHQLKDVKPTPDFVEKLREMMEEIENAINTFKEEQRLIYEELIKEEKTTN
NELSAISRKIDTWALGNSETEKAFRAISSKVPVDKVTPSTLPEEVLDFEKFLQQTGG
RQGAWDDYDHQNFVKVRNKHKGKPTFMEEVLEHLPGKTQDEVQQHEKWYQKF
LALEERKKESIQIWKTKKQQKREEIFKLKEKADNTPVLFHNKQEDNQKQKEEQRK
KQKLAVEAWKKQKSIEMSMKCASQLKEEEEKEKKHQKERQRQFKLKLLLESYTQ
QKKEQEEFLRLEKEIREKAEKAEKRKNAADEISRFQERDLHKLELKILDRQAKEDE
KSQKQRRLAKLKEKVENNVSRDPSRLYKPTKGWEERTKKIGPTGSGPLLHIPHRAI
PTWRQGIQRRV

### Example 6: Screening the CCDC112 monoclonal antibody specifically binding to the antigen

In order to screen the CCDC112 monoclonal antibody specifically binding to the antigen, ELISA was used to detect the concentration-dependent effect of the CCDC112 antibody binding to CCDC112 full-length protein and CCDC112 (73-227) fragment, the latter was used to analyze the binding part (i.e., epitope) of the antibody to CCDC112.

As shown in Fig. 7, with the increase of the concentration of CCDC112 antibody, the value of the ELISA detection results under the condition of CCDC 112 full-length protein coating increased significantly, while the value under the condition of CCDC112 (73-227) fragment coating remained at a low level. This experiment shows that the antibody specifically binds to parts of CCDC112 except for the (73-227) fragment.

Further, the specific binding of the CCDC112 antibody to the antigen was verified by flow cytometry. The HCT116 cell line overexpressing CCDC112 was constructed by stable transfection of liposome, and the cells and the CCDC112 antibody were co-incubated, the unbound antibody was washed away, and then the anti-mouse secondary antibody (FITC modified) was used for flow cytometry and the percentage of cells that stain positive was analyzed.

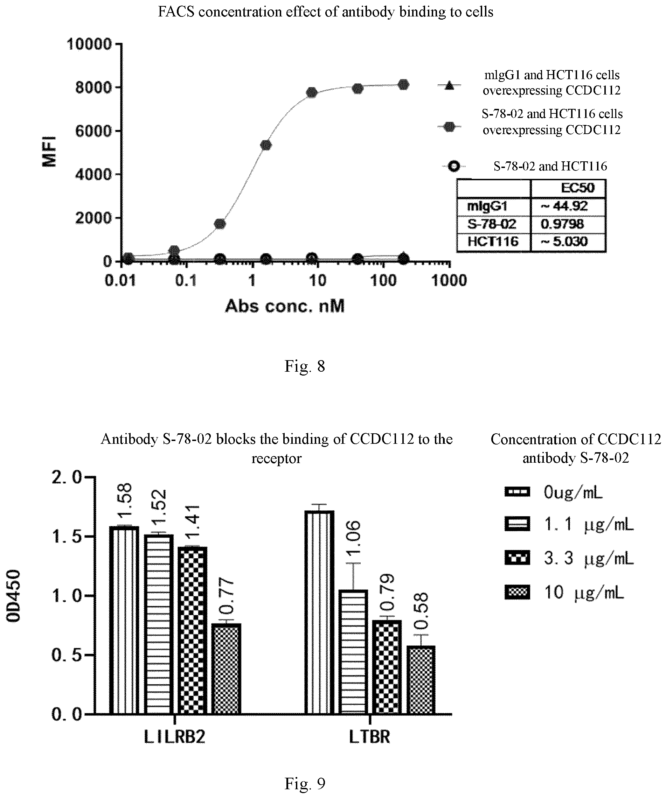

As shown in Fig. 8, the results of flow cytometry indicate that the antibody binds to HCT116 tumor cells instead of CCDC112-knockout HCT116 tumor cells.

### Example 7: Screening the CCDC112 monoclonal antibody that can inhibit the binding of CCDC112 to receptors

In order to screen and identify antibodies that can block the function of CCDC 112, the effect of antibodies on the combination of CCDC112 and its receptors was analyzed by ELISA experiment. Specifically, the CCDC112 recombinant protein was coated on an ELISA plate, and antibodies of different concentrations (0, 1.1, 3.3, 10.0 µg/mL) were added, and the LII,RB2-hFc or LTBR-hFc receptor (containing human antibody Fc fragment) was then added, then anti-human secondary antibody conjugated with horseradish peroxidase was added, and finally the substrate was used for color development, and the absorbance at 450nm was detected to indicate the degree of binding of CCDC112 to the receptor.

As shown in Fig. 9, the antibody shows an inhibitory effect on the binding of CCDC112 to its receptor LILRB2/LTBR, and the degree of inhibition was related to the concentration of the antibody, the higher the concentration, the more significant the degree of inhibition.

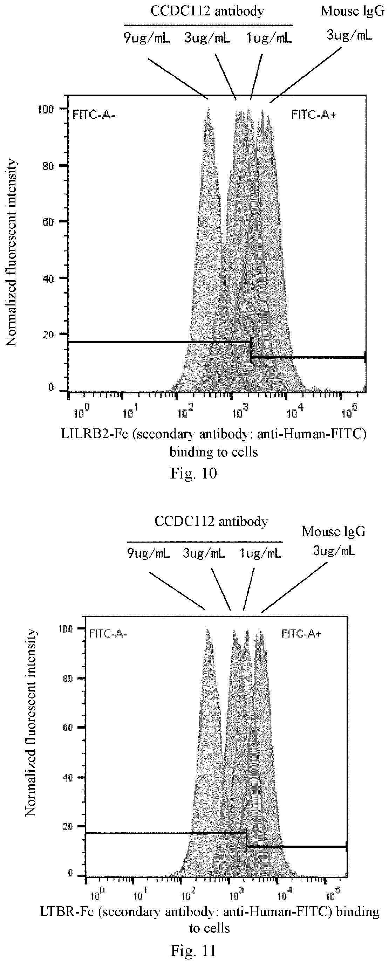

Further, in order to detect the effect of the antibody on the binding of CCDC112 to its receptors (LILRB2, LTBR) on the surface of tumor cells, HCT116 cells stably overexpressing CCDC112 were combined with LELRB2-hFc, and then incubated with different concentrations of CCDC112 antibody or control mouse IgG antibody, and finally detected with FITC-conjugated anti-human secondary antibody. Flow cytometry showed that the CCDC112 antibody inhibited the binding of tumor cells to the CCDC112 receptor (LILRB2), as shown in Fig. 10, and the CCDC112 antibody inhibits the binding of tumor cells to the CCDC112 receptor (LTBR), as shown in Fig. 11.

Routine gene sequencing of the monoclonal antibody molecule hypervariable region was carried out on the screened monoclonal antibody. The obtained nucleotide sequences of LV and HV and the corresponding encoded amino acid sequences, the nucleotide sequence of clone S-78-02 are shown in Table 1. The amino acid sequences are shown in Table 2. Based on the analysis of CDRs of the monoclonal antibody by online software, the amino acid sequences of the CDRs are defined, as shown in Table 3.

**Table 1 Nucleotide sequences of hypervariable regions in monoclonal antibody**

| Monoclonal antibody | VH | VL |
|---|---|---|
| S-78-02 | CAGGTTCAACTGCAGCAGTCTG GACCTGAGCTGGTGAAGCCTGG GGCTTCAGTGAAGATACCCTGCA | GACATCCAGATGAACCAGT CTCCATCTTCCATGTTTGCA TCTCTAGGAGAGAGAGTCA |
| | AGACTTCTGGATACTCATTCACT GACTACAACATAGACTGGGTGA AGCAGGGACTTGGAGAGAGCCT TGAGTGGATTGGAGATATTAATC CTAACTATGGTGGAACTATGTAC AACCAGAAGTTCAAGGGCAAGG CCACATTGACTGTAGACAAGTCC TCCAACACAGCCTACATGGAGCT CCGCAGCCTGACATCTGAAGAC ACTGCAGTCTATTACTGTGCACA TGACGAGTTTATTTACTGGGGCC AAGGGACTCTGGTCACTGTCTCT GCA ( SEQ ID NO: 11 ) | CTATCACTTGCAGGGCGAG TCAGGACATTAATACCTATT TAAGCTGGATCCAGCGGAA ACCAGGGAAATCTCCTAAG ACCCTGATCTATCGTGCAA ACAGATTGGTAGATGGGGT CCCATCAAGGTTCAGTGGC AGTGGATCTGGGCAAGATT ATTCTCTCACCATCAGCAGC CTGGAATATGAAGATATGGG AATTTATTATTGTCTACAGT ATGATGAGTTTCCTCACAC GTTCGGTGGGGGGACCAAG CTGGAAATAAAA (SEQ ID NO: 12) |

**Table 2 Amino acid sequences of VH and VL in monoclonal antibody**

| Monoclonal antibody | VH | VL |
|---|---|---|
| S-78-02 | QVQLQQSGPELVKPGASVKIPCK TSGYSFTDYNIDWVKQGLGESLE WIGDINPNYGGTMYNQKFKGKA TLTVDKSSNTAYMELRSLTSEDTA VYYCAHDEFIYWGQGTLVTVSA (SEQ ID NO: 13) | DIQMNQSPSSMFASLGERVTI TCRASQDINTYLSWIQRKPG KSPKTLIYRANRLVDGVPSR FSGSGSGQDYSLTISSLEYED MGIYYCLQYDEFPHTFGGGT KLEIK (SEQ ID NO: 14) |

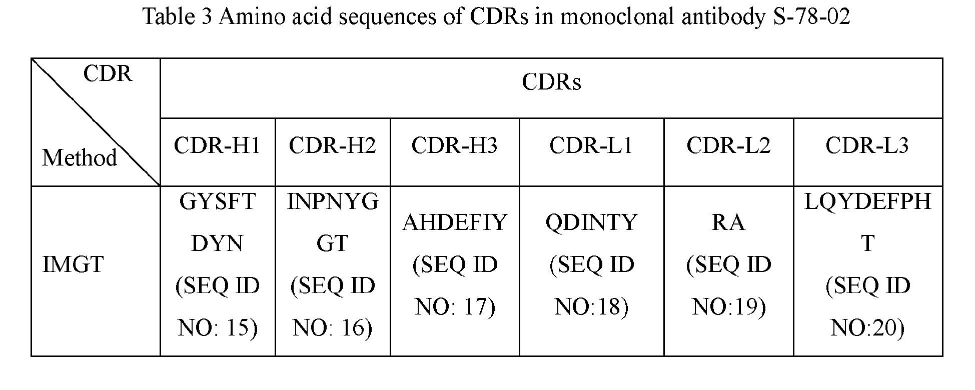

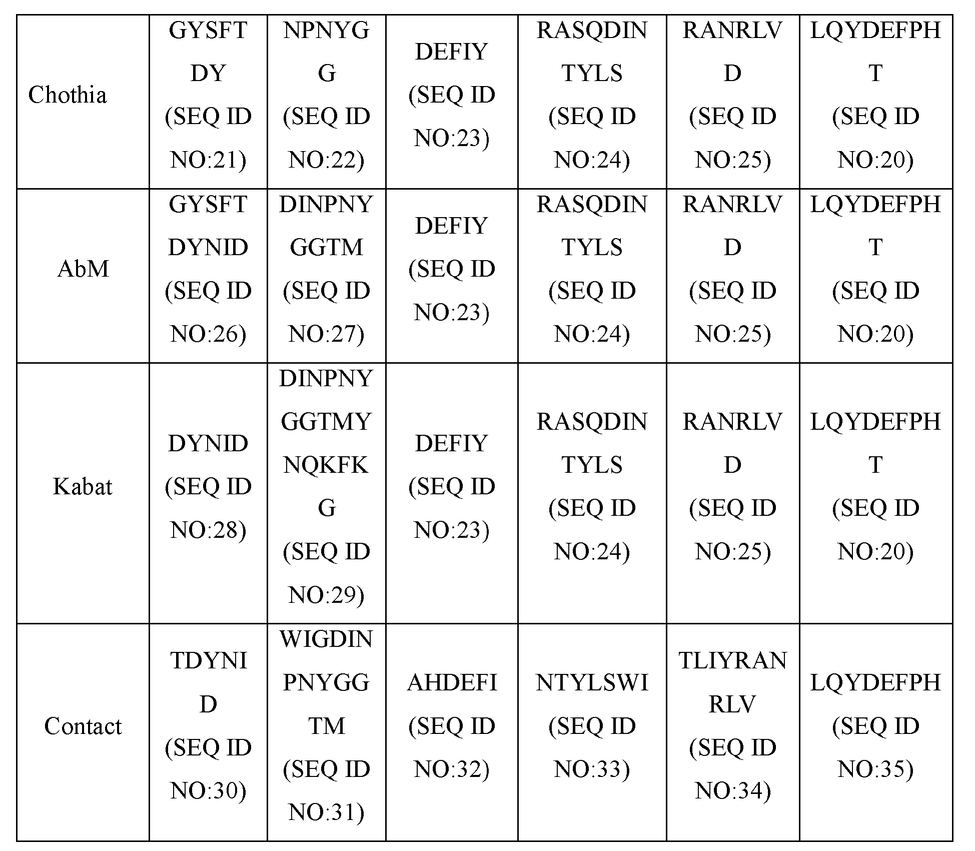

### Example 8: Determination of the binding kinetics of monoclonal antibody and antigen by bio-layer Interferometry

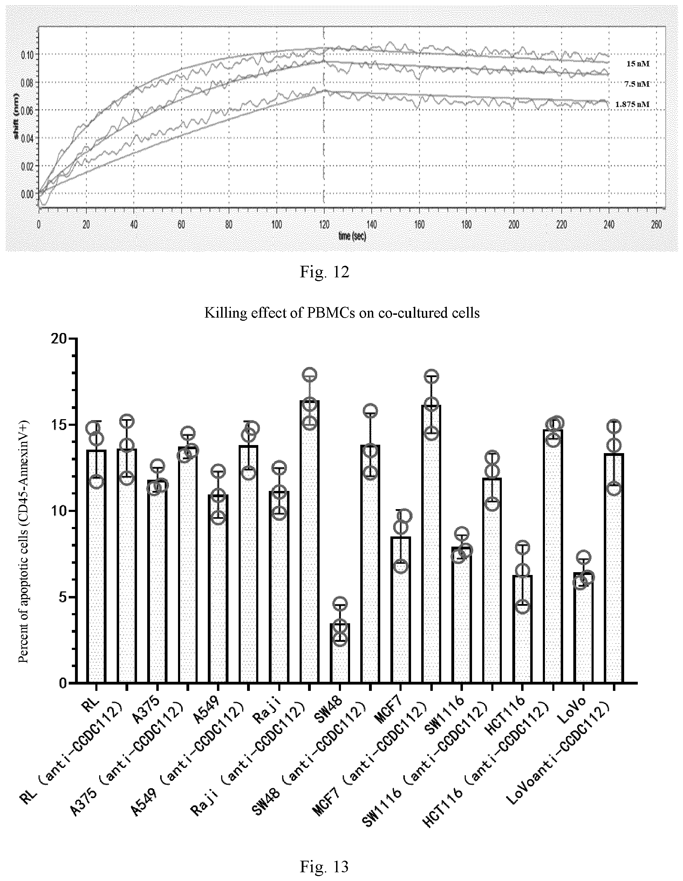

The equilibrium dissociation constant (KD) of the antibody of the invention binding to human CCDC112 was determined using bio-layer interferometry on ProbeLife Gator according to conventional procedures. The antibody (200 nM) was immobilized on the CM5 chip, and the antigen was serially diluted from 15 nM to 1.875 nM for association and dissociation tests. After software analysis, the determined affinity constant was 4.68*10⁻¹⁰M. The affinity-dissociation curve between the monoclonal antibody S-78-02 and CCDC112 is shown in Fig. 12.

### Example 9: In vitro inhibitory effect of CCDC112 monoclonal antibody on tumor cells

In order to identify the inhibitory effect of the CCDC112 antibody (S-78-02) on tumor cells expressing CCDC112, in vitro experiments were carried out as follows: different tumor cells (RL lymphoma cells, A375 melanoma cells, RAJI Lymphoma, MCF7 breast cancer, A549 lung cancer, HCT116, LOVO, SW1116, SW48 colorectal cancer cells) were selected, first incubated with a CCDC112 antibody or control mouse IgG, and then co-cultured for 12h with PBMCs activated by CD3 and CD28 in advance. The apoptotic ratio of CD45- tumor cells was analyzed by Annexin-V-labeled flow cytometry.

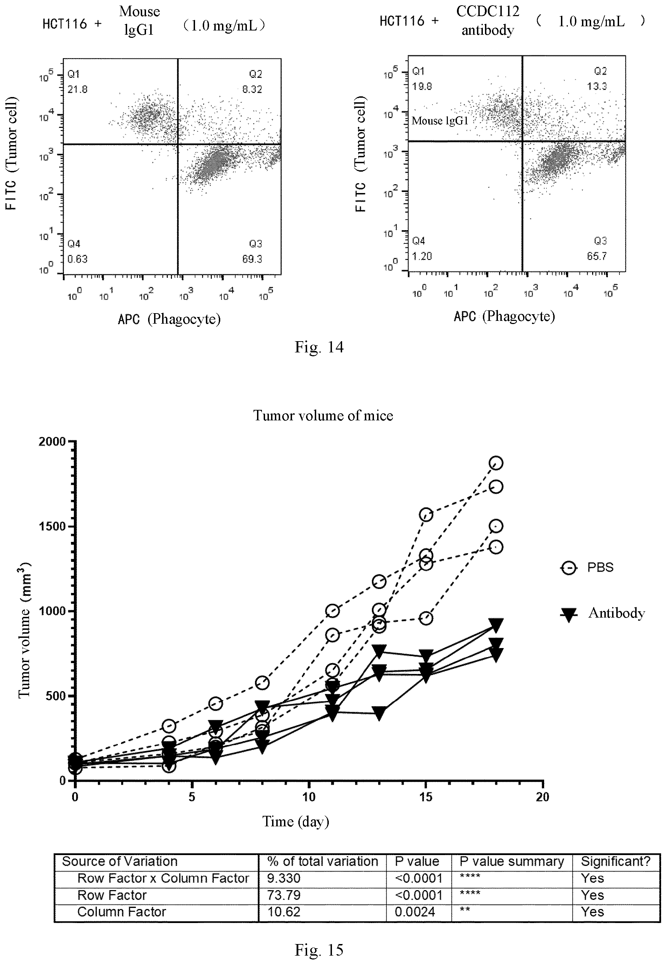

The antibody has different degrees of promoting effect on PBMC killing tumor cells, and the higher the level of CCDC112 expression, the stronger the promoting effect, as shown in Fig. 13. Further, the effect of the antibody was analyzed by macrophage phagocytosis assay. THP1-induced macrophages (APC-labeled) were co-cultured with HCT116 cells (green fluorescent-labeled), and the degree of phagocytosis was analyzed by flow cytometry at 12 hours. The results are shown in Fig. 14, and the CCDC112 antibody increases the phagocytosis of tumor cells by macrophages.

The above results indicate that the expression of CCDC112 is an important selective index for the administration of a CCDC 112 antibody to inhibit tumor cells, and the CCDC112 antibody exerts its effect by specifically inhibiting the function of CCDC112.

### Example 10: In vivo inhibitory effect of CCDC112 monoclonal antibody on tumor cells

In order to identify the inhibitory effect of the CCDC112 antibody on tumor cells expressing CCDC112, different in vivo experiments were carried out as follows:
30 6-8 weeks-old male NPSG mouse models were weighed. Tumor cells expressing CCDC112 at different levels, including HCT116 cells (medium expression), SW48 (strong expression), A549 lung cancer (medium and low expression), MCF7 (medium expression) and RL cells (negative), were cultured in vitro to obtain 1.8×10⁸ cells. After 30 mice were inoculated with PBMCs, they were inoculated with tumor cells on the 3rd day, and then the ratio of hCD45+ cells in blood and body weight of mice were measured once a week. After inoculation, the tumor volume was measured once a week, and the ratio of hCD45+ cells in the mouse blood was measured when the average tumor volume reached about 40-80 mm³. According to the tumor volume and the ratio of hCD45+ cells in the mouse blood, the mice were randomly divided into groups, and then administrated. The start date of administration was considered Day 0. Administration regimen: CCDC112 antibody was injected intraperitoneally at 5 mg/kg three times a week. After the administration started, the tumor growth status of the mice was observed weekly. After the tumor growth, the body weight and tumor volume were measured three times a week, and the relative count of hCD45+ cells in the blood of the mice was monitored by flow cytometry three times a week. When the tumor volume reached the endpoint standard, blood was taken to detect the same indicators as above, and the experiment was ended. The observation of mice included: daily observation. After inoculation, animal morbidity and death were observed every working day. Tumor volume measurement: After inoculation and before grouping, when the tumor was visible, the tumor volume of the experimental animals was measured once a week. After inoculation and grouping, the tumor volume of the animals in the experiment was measured twice a week. Tumor volume measurement is carried out by a two-way measurement method. First, the long and short diameters of the tumor were measured by using a vernier caliper, and then the tumor volume was calculated using the formula TV=0.5*a*b², where a is the long diameter of the tumor and b is the short diameter of the tumor. As shown in the results.

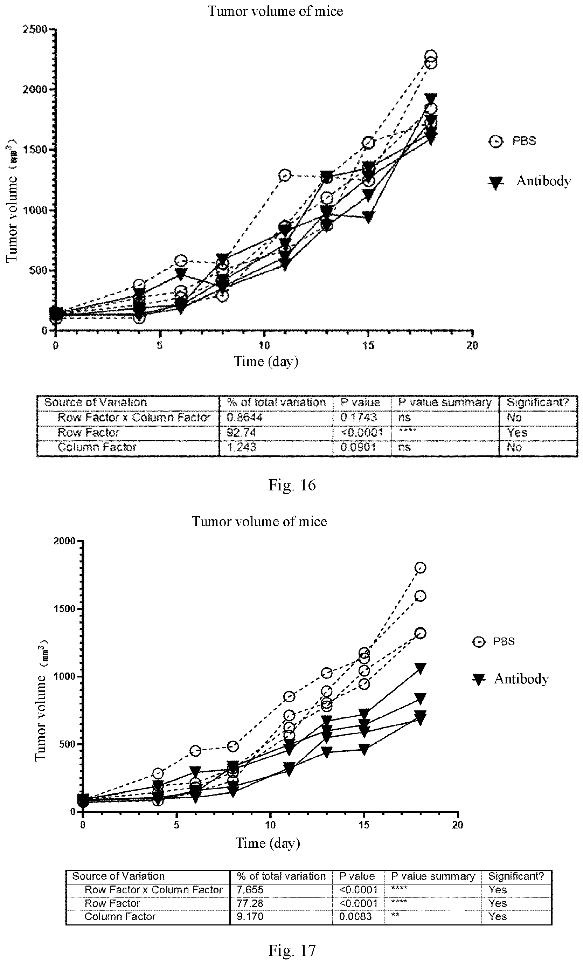

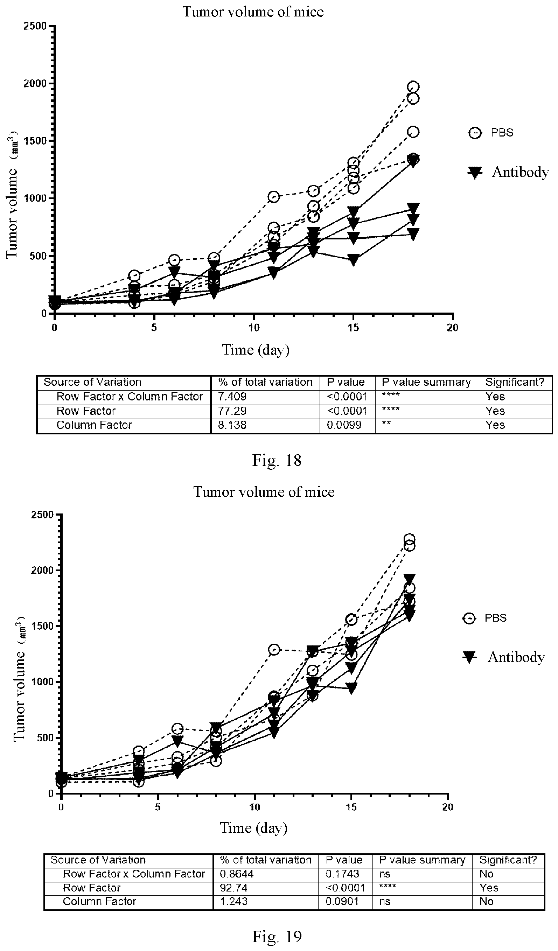

For SW48 colorectal cancer cells that highly express CCDC112, the CCDC112 antibody had a significant inhibitory effect on tumors in mice, and the effect was the strongest among all groups (Fig. 15). For HCT116, A549, and MCF7 tumor cells that moderately express CCDC112, the CCDC112 antibody had a significant inhibitory effect on tumors in mice (Figs. 16-18). For RL lymphoma cells in which CCDC112 was not detected, the CCDC112 antibody had no obvious inhibitory effect on tumors in mice (Fig. 19).

The above results indicate that the analysis of CCDC112 expression is of great significance for the administration of CCDC112 antibody, and tumor cells expressing CCDC112 have a more significant response to the CCDC112 antibody.

The foregoing descriptions of specific exemplary embodiments of the invention have been presented for purposes of illustration and description. These descriptions are not intended to limit the invention to the precise form disclosed, and obviously many modifications and variations are possible in light of the above teaching. The exemplary embodiments were chosen and described in order to explain the specific principles of the invention and its practical application, so that those skilled in the art can carry out and use the various different exemplary embodiments of the invention and various different options and changes. The scope of the present disclosure is intended to be defined by the claims and equivalents thereof.

## Claims

1. A method for regulating tumor cells in vitro or in vivo, wherein said method comprises the following step:
a) for tumor cells expressing CCDC112, altering the function or activity of CCDC112; preferably, the regulating is for inhibiting and the altering is for inhibiting.

2. The method for inhibiting tumor cells in vitro or in vivo according to claim 1, wherein further comprises the following step:
b) contacting the tumor cells with immune cells,
wherein step b) is prior to or subsequent to step a).

3. The method for inhibiting tumor cells in vitro or in vivo according to any one of claims 1-2, wherein the inhibition of the function or activity of CCDC112 is the inhibition at a protein level or at a gene level;
preferably, the methods for the inhibition comprise but are not limited to any one of:
i. editing the CCDC112 gene;
ii. interfering with the expression of the CCDC112 gene; and
iii. inhibiting the function or activity of the CCDC112 protein by an antibody.

4. The method for inhibiting tumor cells in vitro or in vivo according to any one of claims 1-3, wherein the inhibition of the function or activity of CCDC112 results in an effect of inhibiting or blocking the binding of CCDC112 to a receptor LILRB2, LTBR, LILRB4 or CD30.

5. A method for regulating a ratio of MDSC or Treg cells in vitro or in vivo, wherein comprises the step of altering the function or activity of CCDC112 in tumor cells.

6. A method for regulating the activity of LILRB2, LTBR, LILRB4 or CD30 in cells in vitro or in vivo, wherein comprises the step of altering the function or activity of CCDC112 in tumor cells.

7. A method for screening a drug or a reagent for preventing or treating a tumor, wherein by screening an inhibitor or an antibody that inhibits the interaction between CCDC112 and a receptor LILRB2, LILRB4, LTBR or CD30, to obtain a candidate drug or an agent.

8. A method for screening or identifying an inhibitor, wherein comprises any one or more of the following steps:
a) analyzing the effect of a candidate molecule on the binding of CCDC112 to the receptor LILRB2, LTBR, LILRB4 or CD30;
b) analyzing the effect of the candidate molecule on myeloid-derived suppressor cells (MDSCs);
c) analyzing the effect of the candidate molecule on Treg cells; and
d) analyzing the effect of the candidate molecule on immune cells killing tumor cells;
wherein the candidate molecule is a CCDC112 inhibitory molecule;
preferably, the CCDC112 inhibitor is an antibody or antigen-binding fragment thereof.

9. A method for preparing an antibody, wherein comprises:
a) a step of preparing antibodies against CCDC112; and:
b) screening an antibody having an effect in blocking or inhibiting the binding of CCDC112 to the receptor LILRB2, LTBR, LILRB4, or CD30.

10. An agonistic or antagonistic compound, wherein is capable of regulating interaction between CCDC112 and LILRB2, LILRB4, LTBR or CD30; preferably, the compound being a small molecule inhibitor, polypeptide, antibody or antigen-binding fragment thereof.

11. Use of a CCDC112 inhibitor in any one of the following:
a) antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
b) preparation of a product for antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
c) prevention or treatment of a tumor;
d) preparation of a drug for preventing or treating a tumor;
e) regulation of immune response against a tumor;
f) preparation of a drug for regulating immune response against a tumor;
g) inhibiting tumor cell growth in vivo and in vitro; and
h) preparation of a reagent for inhibiting tumor cell growth in vivo and in vitro;
the inhibitor inhibits or blocks the binding of CCDC 112 to the receptor LILRB2, LTBR, LILRB4 or CD30; preferably, the inhibitor is a small molecule inhibitor, polypeptide, antibody or antigen-binding fragment thereof.

12. A method for preventing or treating a tumor, comprising:
contacting immune cells or tumor cells of a subject with an effective dose of CCDC112 antibody, wherein the antibody inhibits the binding of CCDC112 to its receptor LILRB2, LTBR, LILRB4, or CD30;
preferably, the subject has received or is receiving or will receive an additional anticancer therapy;
more preferably, the additional anticancer therapy comprises surgery, radiotherapy, chemotherapy, immunotherapy or hormone therapy.

13. A companion diagnostic method for tumor immunotherapy, wherein comprises:
detecting the expression level or function of CCDC 112 protein in tumor cells of a subject before/after immunotherapy.

14. An isolated antibody or antigen-binding fragment thereof, wherein
a) the antibody or antigen-binding fragment thereof specifically binds to the CCDC112 protein with high affinity; and
b) the antibody inhibits or block the interaction between CCDC112 and any one of LILRB2, LTBR, LILRB4, and CD30;
the antibody additionally has at least one of the following properties:
i. reducing the ratio of immunosuppressive cells, MDSCs;
ii. reducing the ratio of immunosuppressive cells, Treg cells; and
iii. enhancing the killing effect of immune cells (such as PBMCs) on tumor cells.

15. An isolated antibody or antigen-binding fragment thereof, wherein comprises three CDRs in a heavy-chain variable region with the amino acid sequence as set forth in SEQ ID NO: 13 and three CDRs in a light-chain variable region with the amino acid sequence as set forth in SEQ ID NO: 14; or a variant with one or more CDRs each having no more than two amino acid changes relative to the above six CDRs, wherein
preferably, the antibody or antigen-binding fragment thereof comprises the following CDR sequences:
i. HCDR1 having an amino acid sequence as set forth in SEQ ID NO: 15, 21, 26, 38 or 30;
ii. HCDR2 having an amino acid sequence as set forth in SEQ ID NO: 16, 22, 27, 29 or 31:
iii. HCDR3 having an amino acid sequence as set forth in SEQ ID NO: 17, 23 or 32;
iv. LCDR1 having an amino acid sequence as set forth in SEQ ID NO: 18, 24 or 33;
v. LCDR2 having an amino acid sequence as set forth in SEQ ID NO: 19, 25 or 34; and
vi. LCDR3 having an amino acid sequence as set forth in SEQ ID NO: 20 or 35; or
a variant with one or more CDRs each having no more than two amino acid changes relative to the six CDRs i-vi;
more preferably, the antibody or antigen-binding fragment thereof comprises: the heavy-chain variable region comprising the sequences of HCDR1, HCDR2 and HCDR3 defined according to IMGT; and the light-chain variable region comprising the sequences of LCDR1, LCDR2 and LCDR3 defined according to IMGT, wherein
i. the HCDR1 has the amino acid sequence as set forth in SEQ ID NO:15;
ii. the HCDR2 has the amino acid sequence as set forth in SEQ ID NO: 16;
iii. the HCDR3 has the amino acid sequence as set forth in SEQ ID NO: 17;
iv. the LCDR1 has the amino acid sequence as set forth in SEQ ID NO: 18;
v. the LCDR2 has the amino acid sequence as set forth in SEQ ID NO: 19; and
vi. the LCDR3 has the amino acid sequence as set forth in SEQ ID NO: 20; or
a variant with one or more CDRs each having no more than two amino acid changes relative to the six CDRs i-vi;
further preferably, the binding KD of the antibody or antigen-binding fragment thereof to CCDC112 satisfies that KD <2×10⁻⁸;
more preferably, KD <2×10⁻⁸, <1×10⁻⁸, <9×10⁻⁹, <8×10⁻⁹, <7×10⁻⁹, <6×10⁻⁹, <5×10⁻⁹, <4×10⁻⁹, <3×10⁻⁹, <2×10⁻⁹, <1×10⁻⁹ or <1×10⁻¹⁰.

16. The antibody or antigen-binding fragment thereof according to claim 15, comprising a heavy-chain variable region and a light-chain variable region, wherein
a) the amino acid sequence of the heavy-chain variable region comprises an amino acid sequence selected from a group consisting of SEQ ID NO: 13 or has at least 70%, 80%, 90%, 95% or 99% sequence identity to a sequence in the group; and
b) the amino acid sequence of the light-chain variable region comprises an amino acid sequence selected from a group consisting of SEQ ID NO: 14 or has at least 70%, 80%, 90%, 95% or 99% sequence identity to a sequence in the group.

17. The antibody or antigen-binding fragment thereof according to claim 16, wherein the antibody further comprises a conjugation moiety linked to a polypeptide, the conjugation moiety is one or more of a radionuclide, a drug, a toxin, a cytokine, an enzyme, a fluorescein, a carrier protein, a lipid, and a biotin, wherein the polypeptide or antibody and the conjugation moiety is selectively linked through a linker; preferably, the linker is a peptide or a polypeptide;
more preferably, the antibody is selected from a monoclonal antibody, a polyclonal antibody, an antiserum, a chimeric antibody, a humanized antibody and a human antibody; more preferably, the antibody is selected from a multispecific antibody, a single-chain Fv (scFv), a single-chain antibody, an anti-idiotypic (anti-Id) antibody, a diabody, a minibody, a nanobody, a single-domain antibody, a Fab fragment, a F(ab') fragment, a disulfide-linked bispecific Fv (sdFv) and intrabody.

18. An isolated polynucleotide, wherein the polynucleotide encodes the antibody or antigen-binding fragment thereof according to any one of claims 14-17.

19. A recombinant vector, comprising the polynucleotide according to claim 18 and an optional regulatory sequence;
preferably, the recombinant vector is a cloning vector or an expression vector;
more preferably, the regulatory sequence is selected from a leader sequence, a polyadenylation sequence, a propeptide sequence, a promoter, a signal sequence, a transcription terminator, or any combination thereof.

20. A host cell, comprising the recombinant vector according to claim 19;
preferably, the host cell is a prokaryotic cell or a eukaryotic cell.

21. A pharmaceutical composition, comprising one or more of the antibodies or antigen-binding fragments thereof, the polynucleotide, the recombinant vector, and the host cell and the pharmaceutical composition according to any one of claims 14-20;
preferably, the composition further comprises a pharmaceutically acceptable carrier or adjuvant.

22. A kit, comprising one or more of the antibodies or antigen-binding fragments thereof, the polynucleotide, the recombinant vector, the host cell, and the pharmaceutical composition according to any one of claims 14-21 and contained in a suitable container.

23. Use of the antibody or antigen-binding fragment thereof, the polynucleotide, the recombinant vector, the host cell and the pharmaceutical composition according to any one of claims 14-21 in any one of the following:
a) agonizing or antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
b) preparation of a product for agonizing or antagonizing the interaction between CDC112 and LILRB2, LILRB4, LTBR and CD30, wherein preferably, CCDC112 is expressed in tumor cells, and LILRB2, LILRB4, LTBR and CD30 are expressed in immune cells;
c) prevention or treatment of a tumor;
d) preparation of a drug for preventing or treating a tumor;
e) regulation of immune response against a tumor;
f) preparation of a drug for regulating immune response against a tumor;
g) inhibiting tumor cell growth in vivo and in vitro; and
h) preparation of a reagent for inhibiting tumor cell growth in vivo and in vitro.

24. A method for preventing or treating a tumor, comprising:
contacting immune cells and tumor cells from a subject with an effective dose of the antibody or antigen-binding fragment thereof, the polynucleotide, the recombinant vector, the host cell, and the pharmaceutical composition according to any one of claims 14-21;
preferably, before contacting the immune cells and/or tumor cells from the subject with an effective dose of the compound, detecting the expression of CCDC 112 in the tumor cells;
preferably, the immune cells are lymphocytes, more preferably, T lymphocytes;
preferably, the subject has received or is receiving or will receive an additional anticancer therapy;
more preferably, the additional anticancer therapy comprises surgery, radiotherapy, chemotherapy, immunotherapy or hormone therapy.

25. A method for detecting the existence of CCDC 112 in a biological sample in vivo and in vitro, comprising: contacting the biological sample with an effective dose of the antibody or antigen-binding fragment thereof, the polynucleotide, the recombinant vector, the host cell and the pharmaceutical composition according to any one of claims 14-21.

26. A method for inhibiting tumor cell growth, comprising the following steps:
A) analyzing the expression of CCDC112 in tumor cells; and
B) contacting the tumor cells with an antibody that identifies CCDC112; wherein
preferably, the binding KD of the antibody to CCDC112 satisfies that KD <2×10⁻⁸; specifically, KD <2×10⁻⁸, <1×10⁻⁸, <9×10⁻⁹, <8×10⁻⁹, <7×10⁻⁹, <6×10⁻⁹, <5×10⁻⁹, <4×10⁻⁹, <3×10⁻⁹, <2×10⁻⁹, <1×10⁻⁹, or <1×10⁻¹⁰;
or, preferably, the antibody inhibits or blocks the binding of CCDC112 to the receptor LILRB2, LTBR, LILRB4 or CD30; and
C) contacting T lymphocytes, the antibody and the tumor cells with each other.

27. Any one of the preceding claims, wherein the tumor is selected from colorectal cancer, lung cancer, breast cancer, melanoma, lymphoma, liver cancer, head and neck cancer, gastric cancer, kidney cancer, bladder cancer, prostate cancer, testicular cancer, endometrial cancer, breast cancer, and ovarian cancer.
